# EUROPEAN PATENT SPECIFICATION

(11) **EP 4 009 942 B1**
(45) Date of publication and mention of the grant of the patent: **09.08.2023**
(21) Application number: 20751149.4
(22) Date of filing: 06.08.2020
(51) Int. Cl.: A61K 31/46, A61K 9/00, A61P 15/00, A61P 25/22, A61P 25/24, C07D 451/06, A61P 15/10

(54) **COMPOUND FOR COMBINATION TREATMENT**
VERBINDUNG ZUR KOMBINATIONSBEHANDLUNG
COMPOSÉ POUR TRAITEMENT COMBINÉ

(30) Priority: 06.08.2019 EP 19190224
(43) Date of publication of application: 15.06.2022
(60) Divisional application: 23183643.8
(73) Proprietor: Initiator Pharma A/S, 2200 Copenhagen N (DK)
(72) Inventor: SIMONSEN, Ulf, 2200 Copenhagen N (DK); COMERMA-STEFFENSEN, Simon, 2200 Copenhagen N (DK); PETERS, Dan, 2200 Copenhagen N (DK)
(74) Representative: Høiberg P/S
(86) International application number: PCT/EP2020/072092
(87) International publication number: WO 2021/023805

(56) References cited:
- WO-A1-2006/035034
- WO-A1-2008/074798
- US-A1- 2011 207 722
- US-A1- 2014 235 669
- Ichimaru Y ET AL: "5-HT1A-receptor subtype mediates the effect of fluvoxamine, a selective serotonin reuptake inhibitor, on marble-burying behavior in mice", Jpn J Pharmacol., 1 May 1995 (1995-05-01), pages 65-70, XP055814225, DOI: 10.1254/jjp.68.65. PMID: 7494384 Retrieved from the Internet: URL:https://pubmed.ncbi.nlm.nih.gov/749438 4/ [retrieved on 2021-06-15]
- Simonsen Ulf ET AL: "Modulation of Dopaminergic Pathways to Treat Erectile Dysfunction", BASIC & CLINICAL PHARMACOLOGY & TOXICOLOGY, vol. 119, 1 October 2016 (2016-10-01), pages 63-74, XP055814318, COPENHAGEN, DK ISSN: 1742-7835, DOI: 10.1111/bcpt.12653 Retrieved from the Internet: URL:https://api.wiley.com/onlinelibrary/td m/v1/articles/10.1111%2Fbcpt.12653>

## Description

### TECHNICAL FIELD

The present invention relates to a compound for use in the treatment of a combination of erectile dysfunction and depression.

### BACKGROUND

Anxiety and depression (also known as major depressive disorder, clinical depression, and recurrent depressive disorder) are mood disorders that cause fear and worries in anxiety or a persistent feeling of sadness and loss of interest, and the disease affects feelings, thoughts, and behaviour which leads to a variety of emotional and physical illnesses. If left untreated, depression may result in significantly lowered quality of life or even premature death from medical conditions or suicide. A variety of factors including inherited genetic traits, hormonal changes, and physical changes in the brain may contribute to anxiety and depression. Numerous areas of the brain show altered activity in patients with major depressive disorder, and these imbalances in the brain affect specifically the neurotransmitters serotonin, noradrenaline, and dopamine. In line with these observations, the main treatments for depression in the state of the art are drugs, which selectively inhibit the reuptake of serotonin (SSRI), of serotonin and noradrenaline (SNRI), or the degradation of serotonin, noradrenaline, and dopamine by inhibition of monoamine oxidase.

Sexual or erectile dysfunction in men (sometimes also referred to as erectile difficulty, impotence, or male erectile disorder) is the persistent inability to attain and maintain an erection sufficient to allow satisfactory sexual performance. Erection involves different central and peripheral neural and/or humoral mechanisms. Central neurotransmitters and neuropeptides controlling penile erection can either facilitate (e.g. dopamine) or inhibit (e.g. opioid peptides) penile erection by acting in several brain areas. Serotonin can exert both facilitatory and inhibitory effects, depending on the receptor subtype involved. Increased sexual dysfunction is common in major depression, and an explanation is decreased monoamine levels in the brain areas involved in regulation of erectile function. Functional magnetic resonance imaging studies show an overlap of central brain areas (amygdaloid body, dorsal thalamus, hypothalamus, caudate-putamen, cingulate gyrus, insular cortex, visual cortex, sensory cortex, and motor cortex) in depression and sexual dysfunction, which affects the ability to achieve orgasm and sexual desire. Moreover, antidepressants, such as SSRI and SNRI negatively affect the male sexual function (desire-arousal-excitement-orgasm). Although the incidence of sexual dysfunction is lower with some atypical antidepressants including bupropion, mirtazapine, and vortioxetine, compared to SSRI, there is a significant requirement to treat sexual dysfunction induced by antidepressive medication (treatment-emergent erectile dysfunction) also by these drugs. Therefore, a drug that can treat both erectile dysfunction and depression is an unmet need and of a high clinical interest.

Major depressive disorder is one of the main causes of disability worldwide due to its high prevalence and associated impairments. Lifetime prevalence is 14.3% in high-income countries. The Global Burden of Disease study showed a 37.5% burden increase due to major depressive disorder from 1990 to 2010, and major depressive disorder is the second expected leading cause of Disability Adjusted life years in 2020. Anxiety, characterized by excessive fear response and/or worry that interferes with functioning or causes distress has a lifetime prevalence of 16.6% worldwide and woman are twice as much likely to be affected as men. The assessment of treatment outcomes in anxiety and depression has traditionally focused on impairment of mood symptoms. Therefore, SSRI and SNRI are recommended first-line treatment for anxiety and major depressive disorder according to international guidelines. However, patients report that a return to normal level of daily life functioning is more important than symptom-related outcomes. The impairment of functioning may persist after the resolution of mood symptoms, and the persistence of functional disability including sexual dysfunction may increase the risk of experiencing future episodes of major depressive order.

Ejaculation disorders are classified as premature ejaculation and delayed ejaculation, the first defined as lifelong premature ejaculation (less than 1 min ejaculation with vaginal penetration) and acquired premature ejaculation (less than 3 min ejaculation with vaginal penetration). The second defined as two times plus the normal mean of intravaginal ejaculation latency, around 25 min. Ejaculatory disorders will develop from an array of causes as genetic, psychological, iatrogenic (pelvic surgery), and diseases that could affect prolactin and testosterone (diabetes, depression, etc).

Erectile dysfunction is a highly prevalent, age-related global disease estimated to affect 300 million men in 2025. Ejaculatory disorders are mainly represented by premature ejaculation worldwide rates of 5% in men of all ages and 1-4% with delayed ejaculation. In patients with major depressive disorder up to 68% present with comorbid sexual dysfunction, which resolves with antidepressant treatment in only 5% to 30% of patients. Medications of central nervous system disorders with antidepressants also have a negative impact on the erectile function, ability to achieve orgasm, and sexual desire with a predisposition to affect more men than women. Current guidelines recommend on-demand phosphodiesterase type 5 (PDE5) inhibitors, sildenafil, vardenafil, tadalafil, and avanafil as the first line treatment of erectile dysfunction, however approximately 30-40% of men with erectile dysfunction do not respond to PDE5 inhibitor therapy and some men cannot take PDE5-inhibitors due to interactions with common cardiac medications (nitrates). Ejaculation disorders are in lack of treatments. Premature ejaculation is treated with SSRI or dapoxetine. There is no approved treatment for delayed ejaculation. In addition to mood disorders, antidepressants are also used to treat neuropathic pain, therefore sexual dysfunction induced by antidepressants affects a more wide population. When the patients are better treated for their depression and sexual dysfunction, and thereby become healthy, they will typically have a better quality of life.

The current treatment for sexual dysfunction in patients with depression is to use one of the atypical antidepressants as add-on to antidepressive treatment with SSRI or SNRI. An alternative is to change the antidepressive treatment to monotherapy with vortioxetine with the risk that the effect on depression decreases. In patients with mild depression and erectile dysfunction, the recommended treatment consists in phosphodiesterase type 5 inhibitors as add-on to the antidepressive treatment, and in case of vardenafil only 38% of the patients obtained normal erectile function compared to 13% in the placebo group. Therefore, there is still a large group of patients with co-morbidity of depression and erectile dysfunction with unmet need for treatment. However, in the patients with a positive response, there was a correlation of improved erectile function with improvement of the depressive disorder. Bupropion is a noradrenaline-dopamine reuptake inhibitor and nicotinic receptor antagonist, while mirtazapine antagonizes α2-adrenoceptors and serotonin receptors (5-HT₂ and 5-HT₃) and increases dopamine in the prefrontal cortex. Bupropion and mirtazapine are used as add-on to strengthen the antidepressive effect and counteract the devastating effects of other anti-depressive drugs on sexual function. Vortioxetine is an atypical antidepressant, which inhibits serotonin (5-hydroxytryptamine) reuptake and the noradrenaline reuptake as well as agonist on some 5-hydroxytryptamine (5-HT_{1A}, and 5-HT_{1B}) and antagonist on other 5-hydroxytryptamine receptors (5-HT_{1D}, 5-HT₃, and 5-HT₇). Although the incidence of sexual dysfunction is lower in treatment of depressive patients with vortioxetine compared to conventional SSRI e.g. paroxetine and escitalopram, there is significant requirement for treatment of sexual dysfunction induced by antidepressive medication (treatment-emergent sexual dysfunction), also by vortioxetine. Therefore, there is a major unmet need and market for an antidepressant medication, which improves rather than further deteriorates sexual function in patients with depressive disorders.

US 2011/207722 A1 relates to a compound of formula (I), i.e exo-7-(8-H-8-azabicyclo[3.2.1]oct-3-yloxy)-chromen-2-one for use in the treatment of anxiety.

WO 2006/035034 A1 relates to chromen-2-one derivatives as monoamine neurotransmitter re-uptake inhibitors for use in the treatemtn of e.g sexual dysfunction, anxiety or depression and discloses the compound of present formula (I).

WO 2008/074798 A1 relates to chromen-2-one derivatives as monoamine neurotransmitter re-uptake inhibitors for use in the treatment of e.g. sexual dysfunction, anxiety or depression.

US 2014/235669 A1 relates to the use of a monoamine re-uptake inhibitor for use in the treatment of e.g. anxiety, depression or sexual dysfunction.

Ichimaru Y. et al. (Jpn. J. Pharmacol., 1995-05-01, p. 65-70) relates to the effect of fluvoxamine, a selective serotonin reuptake inhibitor, on marble-burying behavior in mice.

Simonsen Ulf et al. (Basic and clinical pharmacology & Toxicology, vol. 119, 2016-10-01, p. 63-74) reviews the possibility of modulating the dopaminergic pathways to treat erectile dysfunction.

### SUMMARY

As outlined above, a compound capable of effecting penile erection while simultaneously treating major depressive disorder, but lacking the adverse effects of typical antidepressants, is highly desired. Specifically, the calming effect of typical SSRIs is undesirable for patients wishing to attain and maintain erection.

The present disclosure thus provides a compound according to formula (I) for use in treatment of major depressive disorder and erectile dysfunction. Particularly, the present disclosure provides a compound according to formula (I) for use in the treatment of major depressive disorder and erectile dysfunction in a patient suffering from both. The inventors have surprisingly found that the compound has effects on erections in rats. The inventors found both duration and frequency of erectile responses to be higher in adults compared to young rats, while the magnitude of the erectile response was higher in young rats compared to the adults. The inventors have surprisingly also found that the erectile response is higher at lower doses, with 0.001 to 0.1 mg/kg doses effecting much more frequent responses than a dose of 1.0 mg/kg. The inventors have surprisingly also found that the compound has an antidepressive effect in two different animal models, namely mouse and rat. These findings demonstrate a completely new way to treat erectile dysfunction and major depressive disorder as a monotherapy using the compound of the present disclosure. In contrast to the state of the art, the compound of the present disclosure is capable of treating both major depressive disorder and erectile dysfunction. These findings could potentially lead to significantly improved quality-of-life for patients suffering from major depressive disorder and erectile dysfunction, major depressive disorder treatment-emergent erectile dysfunction, or erectile dysfunction caused by major depressive disorder.

Thus, according to the invention, a compound of formula (I) is provided: 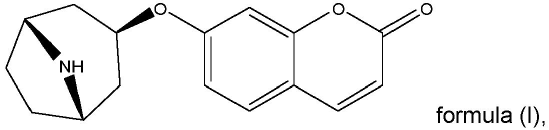 or a pharmaceutically acceptable salt thereof, for use in the treatment, prevention, and/or alleviation of a combination of erectile dysfunction and depression in a mammal.

According to the disclosure, a method is provided for increasing the magnitude of erectile response in a subject in need thereof, the method comprising administering to the subject a therapeutically effective amount of a compound of formula (I) or a pharmaceutically acceptable salt thereof.

According to the disclosure, a method is provided for increasing the duration of an erectile response in a subject in need thereof, the method comprising administering to the subject a therapeutically effective amount of a compound of formula (I) or a pharmaceutically acceptable salt thereof.

According to the disclosure, a method is provided for increasing the frequency of erectile responses in a subject in need thereof, the method comprising administering to the subject a therapeutically effective amount of a compound of formula (I) or a pharmaceutically acceptable salt thereof.

According to the disclosure, a method is provided for inducing relaxation of the corpus cavernosum in a subject in need thereof, the method comprising administering to the subject a therapeutically effective amount of a compound of formula (I) or a pharmaceutically acceptable salt thereof.

According to the disclosure, a method is provided for increasing penile blood flow in a subject in need thereof, the method comprising administering to the subject a therapeutically effective amount of a compound of formula (I) or a pharmaceutically acceptable salt thereof.

### DESCRIPTION OF DRAWINGS

Figure 1: Relationship between plasma (black diamonds) and brain (black circles) concentrations of compound (I) and in vivo DAT transporter occupancy (open bars) in mouse striatum 90 min after p.o. administration of 3, 10 and 30 mg/kg.
Figure 2: Inhibition of in vitro and in vivo WIN binding by compound (I).
Figure 3: WIN in vivo binding time course following 20 mg/kg p.o. in mice.
Figure 4: Effects of Cocaine (black triangles, 25 mg/kg i.p., 0 min), Buprobion (white triangles, 10 mg/kg s.c., 0 min) and compound (I) 30 mg/kg and 3 mg/kg at 0 min (white diamonds, black diamonds) on extracellular DA levels in the N.acc of anesthetized. Extracellular concentrations of DA are expressed as percentage of basal levels of the monoamine in three fractions collected before the drug injection (mean ± S.E.M).
Figure 5: Forced swimming NMRI mice. Compound (I) p.o., t = 60 min.
Figure 6: Mouse tail suspension test. C57 males, 25-28 g, Taconic, 16/8-06. Mice acclimatized to ground floor 5 days before testing. Mice saline injected once a day twice before test day. 22 °C, N = 7-8.
Figure 7: The effects of compound (I) in the nialamide-induced 5-HT syndrome test. 50 mg/kg nialamide s.c. t = 120 min. Compound (I) p.o. t = 0. n = 4.
Figure 8: The effects of compound (I) on locomotor activity. Compound (I) p.o. in NMRI mice. TSE Motility.
Figure 9: Compound (I) (sub-active doses mFST) in combination with WAY100635 (0.1 mg/kg). Compound (I) (p.o. at 60 min) + WAY 100635 (s.c. at 60 min).
Figure 10: Compound (I) (active doses mFST) in combination with WAY100635 (1 mg/kg) in mFST. Compound (I) (p.o. at 60 min) + WAY 100635 (s.c. at 60 min).
Figure 11: Compound (I) (active doses mFST) in combination with SCH23390 in mFST. Compound (I) (p.o. at 60 min) + SCH23390 (s.c. at 60 min).
Figure 12: Compound (I) (active doses) in combination with SCH23390 locomotor activity. Compound (I) p.o. + SCH23390 s.c.
Figure 13: Marble burying. In the marble-burying test mice exposed to a novel object (marbles) bury them in the sawdust floor covering. Compounds showing anxiolytic-like activity decrease the number of buried marbles at non-sedative doses. Compound (I) (IP2018) was administered per oral to NMRI mice (n=8), and shows dose-dependently (0.1-1.0 mg/kg) increases in the number of visible marbles suggesting an anxiolytic effect. The number of marbles covered by sawdust was counted at the end of a 60-min session.
Figure 14: Stress-induced hyperthermia. When mice are confronted with a stress-full event, the body temperature rises. Vehicle and compound (I) (IP2018, 0.1 mg/kg, 1 mg/kg, and 3 mg/kg) was administered per oral to NMRI mice (n=8 in each group), and did not change body temperature in resting conditions (upper panel). Exposure of the mice to stress elevated the body temperature in vehicle-treated mice, while the temperature rise was significantly prevented in compound (I)-treated mice at all three doses (lower panel).
Figure 15: Effects of Compound I in the mouse zero maze . Mice were treated (peroral administration) for 7 days with either, vehicle, duloxetine (10 mg/kg), and compound I (0.1 mg/kg, 1 mg/kg, and 3 mg/kg) (n=8 in each group). The effect of 3 mg/kg compound I was similar to the effect of 10 mg/kg duloxetine in the mouse zero maze test.

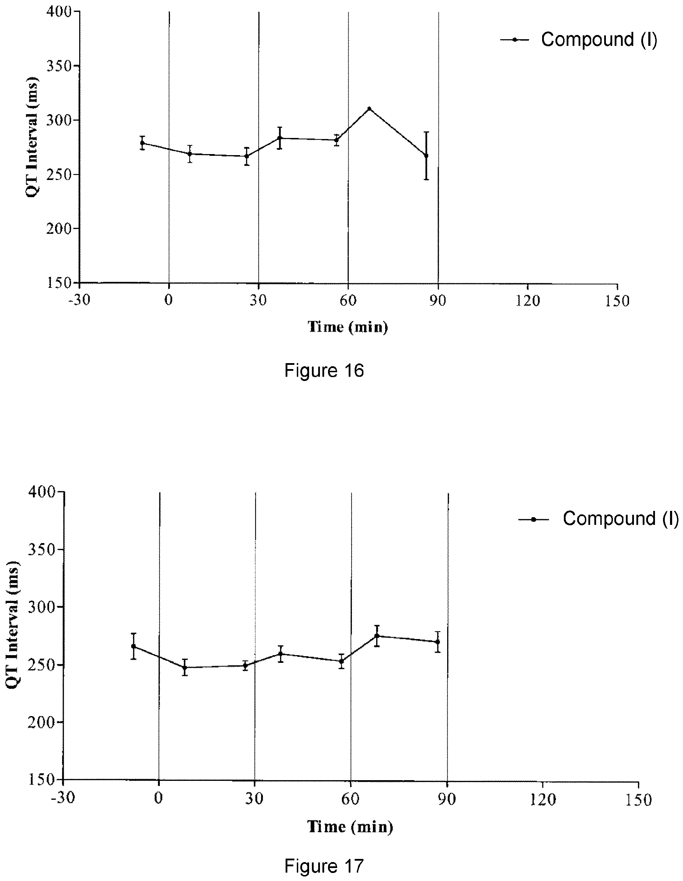

Figure 16: Effects of compound (I) on QT interval pacing data (90 bpm) in anaesthetised beagle dogs. Data presented are mean ± s.e. mean where n = 4 for all time points, excluding D2t7 where n = 3, D3t7 where n = 2, and D4t7 and D4t26 where no data was available. Animals received 1, 3, 10, and 30 mg/kg compound (I) as 4 intravenous infusions (starting at t0, D1t30, D2t30 and D3t30, respectively). Each dose was administered over 5 min, using an infusion pump, and the dose volume for each treatment was 2 ml/kg, with the exception of the fourth dose which was 3 ml/kg. The vertical lines represents the start of each dose infusion.
Figure 17: Effects of compound (I) on QT interval pacing data (110 bpm) in anaesthetised beagle dogs. Data presented are mean ± s.e. mean where n = 4 for all time points, excluding D4t7 and D4t26 where no data was available. Animals received 1, 3, 10, and 30 mg/kg compound (I) as 4 intravenous infusions (starting at t0, D1t30, D2t30 and D3t30, respectively). Each dose was administered over 5 min, using an infusion pump, and the dose volume for each treatment was 2 ml/kg, with the exception of the fourth dose which was 3 ml/kg. The vertical lines represents the start of each dose infusion.
Figure 18: Plasma PK profiles of compound (I) in mouse plasma.
Figure 19: Plasma PK profiles of compound (I) in rat plasma.
Figure 20: Plasma PK profiles of compound (I) in dogs.
Figure 21: Compound (I) dosed to mouse p.o. Dose versus AUC is showing a slight tendency to sublinear kinetics.
Figure 22: Compound (I) dosed to rat s.c. Dose versus AUC is showing linear kinetics.
Figure 23: Compound (I) dosed p.o. to mouse. Plasma concentration versus dose at steady state.
Figure 24: Compound (I) dosed p.o. to rat. Plasma concentration versus dose at steady state.
Figure 25: Average spontaneous increases in (A) intracavernous pressure, (B) duration and (C) frequency of these events after infusion of the compound (I) (1 mg/kg) in young (6 week old male Wistar rats) and old (14-16 week old male rats) rats. Results are means ±s.e. mean.
Figure 26: Average rises in number of spontaneous erections measured as intracavernous pressure (ICP) after infusion of, respectively, 0.001 mg/kg, 0.01 mg/kg, 0.1 mg/kg, and 1 mg/kg compound (I) in anesthetized rats (n=4-6).

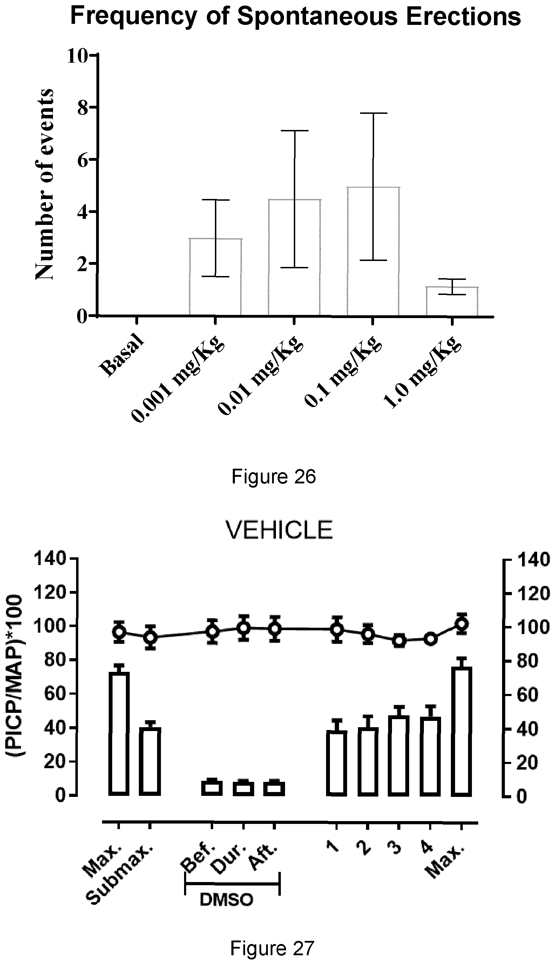

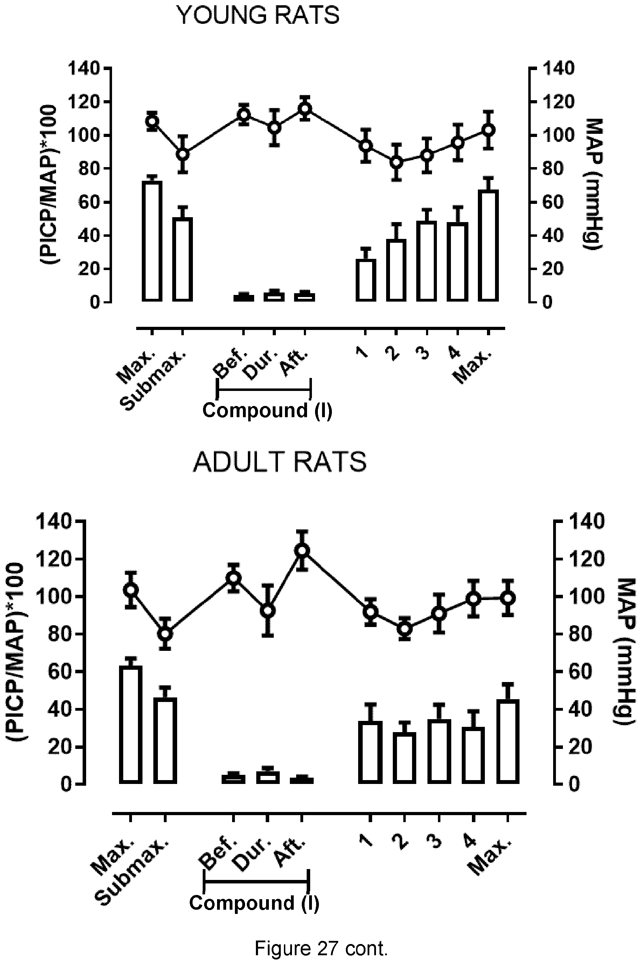

Figure 27: Average effect of (A) vehicle (n=5), and compound (I) on mean arterial pressure (MAP) and erectile response measured as peak intracavernous pressure (PICP) over MAP (PICP/MAP) in anesthetized (B) 6 week old male Wistar rats (n=6), and (C) 14-16 week old male rats (n=6). Vehicle and compound (I) were administered after establishing the maximal (10 Hz, 1 ms, 6 V) and submaximal (submax) responses (10 Hz, 1 ms, 0.6 - 1.55 V) to cavernous nerve stimulation for 30 s. Submaximal stimulation was repeated 3, 13, 23, and 33 min after drug or vehicle administration. Upper traces show the mean arterial pressure (MAP), bars represent the erectile response ((PICP/MAP)·100), where PICP is the peak intracavernous pressure.

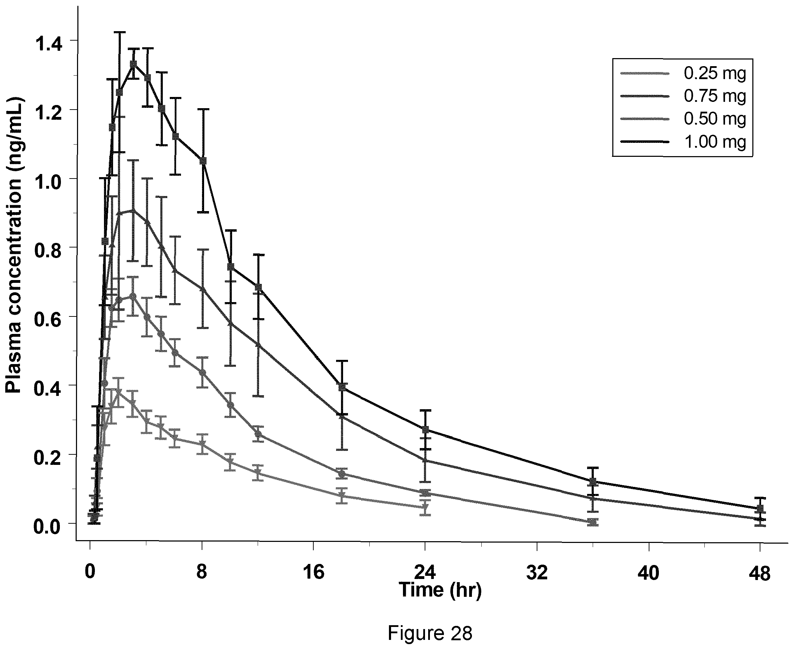

Figure 28: Plasma concentrations of compound (I) over time after per oral administration of compound (I) in phase I trial in man. The Figure shows the plasma concentrations after administration of 0.25 mg/kg, 0.5 mg/kg, 0.75 mg/kg, and 1 mg/kg in healthy volunteers.
Figure 29: Original traces showing the effect of compound (I) on resting tone in rat corpus cavernosum strips. Increasing concentrations (10⁻⁹-3×10⁻⁵ M) of compound (I) were added. Compound (I) induced small relaxations, while at the highest concentration (3×10⁻⁵ M) contraction was observed occasionally (lower trace). The traces shows Compound (I) responses in the presence of vehicle, sildenafil (10⁻⁷ M), guanethidine (10⁻⁵ M), and L-NOARG (10⁻⁴ M).
Figure 30: Average results showing the effect of compound (I) on resting tone in rat corpus cavernosum strips. Increasing concentrations (10⁻⁹-3×10⁻⁵ M) of compound (I) were added. Compound (I) induced small relaxations, while at the highest concentration (3×10⁻⁵ M) contraction was observed occasionally (lower trace). Compound (I) responses were obtained in the presence of vehicle, sildenafil (10⁻⁷ M), guanethidine (10⁻⁵ M), and L-NOARG (10⁻⁴ M). Results are means±s.e. mean of preparations from 5 rats.
Figure 31: Original traces showing relaxations to compound (I) in rat corpus cavernosum strips contracted with phenylephrine (Phe). Representative traces show compound (I) relaxation in the presence of vehicle, guanethidine (10⁻⁵ M), L-NOARG (10⁻⁴ M), sildenafil (10⁻⁷ M), in phenylephrine-contracted preparations.
Figure 32: Average relaxations induced by compound (I) in rat corpus cavernosum strips. In phenylephrine-contracted preparations, increasing concentrations (10⁻⁹-3×10⁻⁵ M) of compound (I). Compound (I) relaxations were obtained in the presence of vehicle, guanethidine (10⁻⁵ M), sildenafil (10⁻⁷ M), and L-NOARG (10⁻⁴ M). Results are means±s.e. mean of preparations from 5 rats. *P<0.05, compared to the control curve for compound (I).
Figure 33: Flow laser doppler raw data from mice erectile function (top), and average flow changes (EF) in mice erectile tissue (bottom). The increase in penile flow induced by compound (I) (1 mg/kg) was highly significant and increased 4.99 times, n=2.

### DETAILED DESCRIPTION

The present disclosure relates to a compound of formula (I) (compound (I)) for use in the treatment of a disease or disorder, or a group of diseases or disorders, involving erectile dysfunction and depression. Treatment of such diseases or disorders by administration of said compound is herein demonstrated. More specifically, it is demonstrated that compound (I) acts as an antidepressant in mice and rats. It is also demonstrated that compound (I) induce erection in rats, and has anxiolytic effects.

### Definitions

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

The term "pharmaceutically acceptable salt" of a compound refers to a salt that is pharmaceutically acceptable, as defined herein, and that possesses the desired pharmacological activity of the parent compound. Pharmaceutically acceptable salts include acid addition salts formed with inorganic acids, e.g. hydrochloric acid, hydrobromic acid, sulfuric acid, nitric acid, phosphoric acid; or formed with organic acids, e.g. acetic acid, benzenesulfonic acid, benzoic acid, camphorsulfonic acid, citric acid, ethanesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2-naphthalenesulfonic acid, propionic acid, salicylic acid, succinic acid, tartaric acid, p-toluenesulfonic acid, trimethylacetic acid; or salts formed when an acidic proton present in the parent compound either is replaced by a metal ion, e.g., an alkali metal ion, an alkaline earth ion, or an aluminum ion; or coordinates with an organic or inorganic base. Acceptable organic bases include e.g. diethanolamine, ethanolamine, N-methylglucamine, triethanolamine, morpholine, and tromethamine. Acceptable inorganic bases include e.g. ammonia, aluminum hydroxide, calcium hydroxide, potassium hydroxide, sodium carbonate and sodium hydroxide.

"Therapeutically effective amount" means an amount of a compound that, when administered to a subject for treating a disease state, is sufficient to effect such treatment for the disease state. The "therapeutically effective amount" will vary depending on the disease or disorder state being treated, the severity of the disorder treated, the age and relative health of the subject, the route and form of administration, the judgment of the attending medical or veterinary practitioner, etc.

As used herein the terms "treatment" or" treating" is an approach for obtaining beneficial or desired results including clinical results. Beneficial or desired clinical results can include, but are not limited to, alleviation or amelioration of one or more symptoms or conditions, diminishment of extent of disease or disorder, stabilized (i.e., not worsening) state of disease or disorder, prevention of the disease or disorder, delay or slowing of disease or disorder progression, amelioration or palliation of the disease state, and remission (whether partial or total) whether detectable or undetectable.

Whenever a chiral carbon is present in a chemical structure, it is intended that all stereoisomers associated with that chiral carbon are encompassed by the structure, unless otherwise specified. Using the Cahn-Ingold-Prelog RS notational system, any asymmetric carbon atom may be present in the (R)- or (S)-configuration, and the compound may be present as a mixture of its stereoisomers, e.g. a racemic mixture, or one stereoisomer only. For bicyclic moieties disclosed herein, substituents may be attached in an *endo* configuration, an exo configuration, or both. It is intended that all stereoisomers associated with that bicyclic moiety are encompassed by the structure, unless otherwise specified.

The compound of the invention may exist in a tautomeric form. Any such tautomer is considered to be within the scope of the invention.

Also, in the compound of formula (I) as defined herein, any hydrogen atom may be replaced by a deuterium (²H), and any such deuterated compound of formula (I), comprising one or more deuterium atoms in place of the corresponding number of hydrogen atoms, is considered to be within the scope of the invention.

The terms "IP2018", "compound (I)", and "compound of formula (I)" are used synonymously herein.

### Compound for use

In one embodiment of the present disclosure, a compound of formula (I) (compound (I)) is provided, 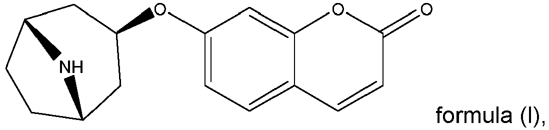 or a pharmaceutically acceptable salt thereof, for use in the treatment, prevention, or alleviation of a combination of erectile dysfunction and depression. The studies outlined in the examples section revealed a positive effect of the compound on erectile function in rats, while the use of the compound for treatment of depression has been established.

In one embodiment of the present disclosure, the compound of formula (I) or a pharmaceutically acceptable salt thereof is used for treatment, prevention, or alleviation of erectile dysfunction caused by depression.

In one embodiment of the present disclosure, the compound of formula (I) or a pharmaceutically acceptable salt thereof is used for treatment, prevention, or alleviation of erectile dysfunction and depression, wherein the erectile dysfunction is treatment-emergent erectile dysfunction. By treatment-emergent erectile dysfunction is meant an erectile dysfunction where the erectile dysfunction is caused by the treatment of another disease, disorder, or condition.

In one embodiment of the present disclosure, the treatment-emergent erectile dysfunction is drug-induced erectile dysfunction, wherein a medicament used for treatment of another disease, disorder, or condition in the subject causes erectile dysfunction as an adverse effect. An adverse effect of the medicament is for instance psychological in nature, such as lowered libido, which can lead to difficulties achieving or maintaining erection. Alternatively, the adverse effect of the medicament is physiological in nature, such as hormonal, which can lead to difficulties achieving or maintaining erection. It is also considered that psychological aspects of a mental disorder, such as feelings of sadness, anxiety, or indifference, can lower the libido of the person suffering from depression, thus affecting the person's ability to attain and maintain erection.

In one embodiment, the present invention relates to treatment of depression and erectile dysfunction, depression treatment-emergent erectile dysfunction, or erectile dysfunction caused by depression, by administration of the compound of formula (I) to a subject in need thereof.

In one embodiment of the present disclosure, the compound of formula (I) or a pharmaceutically acceptable salt thereof is used for treatment, prevention, or alleviation of erectile dysfunction, where the erectile dysfunction is treatment-emergent erectile dysfunction caused by a medicament which is an antidepressant, an NSAID, finasteride, an antiepileptic, or a neuroleptic.

In one embodiment of the present disclosure, the compound of formula (I) or a pharmaceutically acceptable salt thereof is used for treatment, prevention, or alleviation of erectile dysfunction, wherein the erectile dysfunction is treatment-emergent erectile dysfunction caused by a medicament which is an antidepressant.

In one embodiment of the present disclosure, the compound of formula (I) or a pharmaceutically acceptable salt thereof is used for treatment, prevention, or alleviation of erectile dysfunction, wherein the erectile dysfunction is treatment-emergent erectile dysfunction caused by treatment of depression with an antidepressant. In one embodiment, the antidepressant is for instance an SSRI or an SNRI. Because the compound of formula (I) is a dual serotonin/dopamine reuptake inhibitor, the compound is useful in the treatment of both the erectile dysfunction and the depression. Thus, in one embodiment treatment with said antidepressant is stopped, and the compound of formula (I) is instead administered to treat both the erectile dysfunction and the depression. In yet another embodiment, treatment with the antidepressant is not stopped, but the compound of the present disclosure is administered to alleviate the symptoms of the treatment-emergent erectile dysfunction caused by the antidepressant.

In one embodiment of the present disclosure, the compound of formula (I) or a pharmaceutically acceptable salt thereof is used for treatment, prevention, or alleviation of erectile dysfunction and depression caused by said erectile dysfunction. The compound is considered especially useful for the treatment of depression caused by erectile dysfunction, as it is a dual serotonin/dopamine reuptake inhibitor.

In one embodiment, the compound of formula (I) or a pharmaceutically acceptable salt thereof is used for treatment, prevention, or alleviation of a combination of erectile dysfunction and depression.

In one embodiment of the present disclosure, the compound of the present disclosure is administered to a subject in need thereof to treat a combination of erectile dysfunction and depression. In a preferred embodiment, the subject is a mammal. In a further preferred embodiment, the mammal is human. In an even more preferred embodiment, the human is male.

The compound of formula (I) is a dual serotonin/dopamine reuptake inhibitor. Accordingly, in one embodiment of the disclosure, the compound of formula (I) is used for treatment, prevention, or alleviation of a combination of erectile dysfunction and depression. The compound of formula (I) is especially useful for treatment, prevention, or alleviation for any of the combinations of the above diseases or disorders because it is a dual serotonin/dopamine reuptake inhibitor.

The compound of the disclosure may be comprised within a pharmaceutical composition. The composition may be administered by any convenient route, which suits the desired therapy. Preferred routes of administration include oral administration, in particular in tablet, in capsule, in dragee, in powder, or in liquid form, and parenteral administration, in particular cutaneous, subcutaneous, intramuscular, or intravenous injection.

### Medical use

As shown in the examples herein below, the compound of formula (I) is useful for the treatment of diseases or disorders such as erectile dysfunction and depression. Accordingly, one embodiment of the present disclosure provides for a therapeutically effective amount of the compound of formula (I) for use in the treatment, prevention, or alleviation of a combination of erectile dysfunction and depression, in a subject in need thereof.

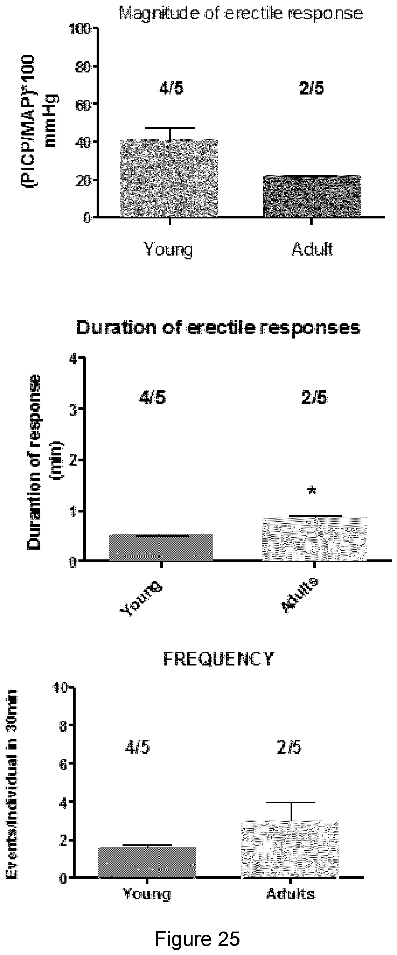

As shown in Example 25, compound (I) induces erection in both young and adult rats. As outlined in figure 25, compound (I) is capable of increasing the magnitude of the erectile response, increase the duration of the erectile response, and/or increase the frequency of the erectile response. Accordingly, one embodiment of the present disclosure provides for a method for increasing the magnitude of erectile response in a subject in need thereof, the method comprising administering to the subject a therapeutically effect amount of a compound of formula (I). The increased magnitude of the erectile response was especially pronounced in young test animals. Thus, in a further embodiment of the present disclosure, the subject is a young male. In a specific embodiment of the disclosure, the male is below 40 years old, such as below 35 years old, such as below 30 years old, such as below 25 years old, such as below 20 years old. Another embodiment of the disclosure provides for a method for increasing the duration of an erectile response in a subject in need thereof, the method comprising administering to the subject a therapeutically effective amount of a compound of formula (I). The increased duration of the erectile response was particularly pronounced in adult test animals. Thus, in a further embodiment of the present disclosure, the subject is an adult male. In a specific embodiment, the subject is an adult male over the age of 20, such as an adult male over the age of 25, such as an adult male over the age of 30, such as an adult male over the age of 35, such as an adult male over the age of 40, such as an adult male over the age of 45, such as an adult male over the age of 50, such as an adult male over the age of 55, such as an adult male over the age of 60, such as an adult male over the age of 65, such as an adult male over the age of 70, such as an adult male over the age of 75. Another embodiment of the present disclosure provides for a method for increasing the frequency of erectile responses in a subject in need thereof, the method comprising administering to the subject a therapeutically effect amount of a compound of formula (I). The increased frequency of erectile responses was especially pronounced for adult test animals. Accordingly, in a further embodiment of the present disclosure, the subject is an adult male. In a specific embodiment, the subject is an adult male over the age of 20, such as an adult male over the age of 25, such as an adult male over the age of 30, such as an adult male over the age of 35, such as an adult male over the age of 40, such as an adult male over the age of 45, such as an adult male over the age of 50, such as an adult male over the age of 55, such as an adult male over the age of 60, such as an adult male over the age of 65, such as an adult male over the age of 70, such as an adult male over the age of 75.

As shown in Example 27, compound (I) can induce relaxation in the corpus cavernosum. Accordingly, in one embodiment of the present disclosure, a method is provided for inducing relaxation of the corpus cavernosum in a subject in need thereof, the method comprising administration to the subject a therapeutically effective amount of a compound of formula (I).

As shown in Example 28, administration of compound (I) to mice increased the penile blood flow in the test animals. Thus, one embodiment of the present disclosure provides for a method for increasing penile blood flow in a subject in need thereof, the method comprising administering to the subject a therapeutically effective amount of a compound of formula (I).

Any of the uses or methods described above for the treatment of erectile dysfunction and/or related side-effects such as reduced magnitude of erectile response, reduced duration of erectile response, reduced frequency of erectile response, impaired relaxation of the corpus cavernosum, or reduced penile blood flow may be further useful for the treatment of a subject suffering from erectile dysfunction and/or the above-mentioned relates side-effects in combination with depression. Thus, one embodiment of the present disclosure provides the Compound of formula (I) for use in a method for treating, preventing, or alleviating erectile dysfunction, for increasing the magnitude of erectile response, for increasing the duration of an erectile response, for increasing the frequency of erectile responses, for inducing relaxation of the corpus cavernosum, and/or for increasing penile blood flow in a subject in need thereof, wherein the subject further suffers from depression.

### EXAMPLES

### Example 1: Compound stability

A purity and stability study was performed on exo-7-(8-aza-bicyclo[3.2.1]oct-3-yloxy)-chromen-2-one (compound (I)) hydrochloride anhydrate, showing that the compound is chemically and thermodynamically stable as solid and in solution, but sensitive to light when in solution. The results are summarised in Table 1.

**Table 1. Stability results for exo-7-(8-aza-bicyclo[3.2.1]oct-3-yloxy)-chromen-2-one. m/z values found by API-ES (positive), h = hours, d = days, m = months, ND = Not Detected.**

| | **Test** | **Result** | **Remark** |
|---|---|---|---|
| | **Purity HPLC** | | |
| | Area%, compound (I) | 99.5 | |
| | Area%, unidentified (RT 2.9 min) | 0.05 | |
| | Area%, unidentified (RT 10.7 min) | 0.36 | |
| **Stability at pH 1.0** | | 100 ND | 0.15 mM |
| | Compound (I), % left after 1 d at 40°C | | |
| | Degradation products ≥ 0.05 area% | | |
| **Stability at pH 7.4** | | 100 BD | 0.15 mM |
| | Compound (I), % left after 1 d at 40°C | | |
| | Degradation products ≥ 0.05 area% | | |
| **Photostability at pH 7.4** | | 11 | Compound (I) is very sensitive to light. Solutions should be protected from light. |
| | Compound (I), % left after 3 h at 250 W/m² | | |
| Degradation products ≥ 5.0 area% | | | |
| | Unidentified (RT 1.7 min) | 6.9 | |
| | *m*/*z* 290 (RT 2.3 min) | 31.5 | |
| | *m*/*z* 290 (RT 3.5 min) | 11.4 | |
| | *m*/*z* 248 (RT 8.8 min) | 8.1 | |
| **Oxidation, 5 % H₂O₂** | | 83 | |
| | Compound (I), % left after 1 d at 40°C | | 0.15 mm, |
| Degradation products ≥ 1.0 area% | | | Compound (I) is sensitive to oxidation. |
| | Unidentified (RT 2.3 min) | 1.2 | |
| | *m*/*z* 288 (RT 12.9 min) | 2.6 | |
| Solid state stability | | 100 ND | Compound (I) is stable in the solid state when stored for 26 days at 40 °C / 75 % RH. |
| | Compound (I), % left after 26 d at 40 °C 75 | | |
| | % rel. humidity in open container. | | |
| | Degradation products ≥ 0.05 area% | | |

### Conclusion

Compound (I) shows good stability in solution over a range of pH values but is sensitive to oxidation and to light. In the solid state, compound (I) is stable over 26 days.

### Example 2: In vitro potency

Compound (I) has been characterized as a monoamine reuptake inhibitor, based on the profile in a series of functional assays (Table 2). A good correlation between human and rat systems was observed in binding assays for 5-HT but to a lesser extend for NA transporters (Table 3).

**Table 2. Monoamine reuptake inhibition by compound (I).**

| **Transporter** | **³H-uptake rat IC₅₀ (nM)** | **³H-uptake human IC₅₀ (nM)** | **pK_{I} on human transporter** | **Human transporter ratio** |
|---|---|---|---|---|
| 5-HT | 0.45 | 1.8 | 8.7 | 1 |
| NA | 85 | 46 | 7.3 | 26 |
| DA | 260 | 100 | 7.0 | 56 |

**Table 3. Binding assays for citalopram and nisoxetine.**

| **³H-Citalopram binding Kᵢ (nM)** | | **³H-Nisoxetine binding Kᵢ (nM)** | |
|---|---|---|---|
| Rat | Human | Rat | Human |
| 0.28 | 0.24 | 1,300 | 270 |

### Conclusion

In vitro, Compound (I) is a potent inhibitor of 5-HT transporters with a 26 and a 56 fold lower affinity for human NA and DA transporters, respectively. The 5-HT:NA:DA ratio, however, is dependent on the assay system used (see also Table 4).

### Example 3: In vitro selectivity

In the MDS LeadProfilingScreen (consisting of 67 different receptors, transporters and ion channels) supplemented with a range of in-house nicotinic binding assays, compound (I) was found to be rather selective for the relevant molecular targets with a few exceptions showing more than 50% inhibition at 10 µM (Table 4). IC₅₀ or K_{I} values were subsequently determined for targets where >60 % inhibition was observed in the initial screen.

**Table 4: In vitro selectivity of compound (I).**

| **Transporter/Receptor** | **Species** | **% Inhibition at 10 µM** | **IC₅₀ (nM)** | **K_{I} (nM)** |
|---|---|---|---|---|
| SERT | Human | 103 | | 0.37 |
| NET | Human | 92 | | 594 |
| DAT | Human | 97 | | 155 |
| Nicotinic α₃β₄ | Human | 57 | | 5,050 |
| Nicotinic α₄β₂ | Rat | | 31,000 | |
| Nicotinic α₇ | Rat | | >10,000 | |
| Serotonin 5-HT₃ | Human | 86 | | 1,040 |
| Sigma σ₁ | Human | 59 | | |

Compound (I) showed affinity in the micromolar range for nicotinic acetylcholine receptors and for 5-HT₃ receptors (Table 5).

**Table 5. Affinity of compound (I) to selected off-targets**

| **Receptor** | **Kᵢ (nM)** | **Separation to transporter (Kᵢ, nM)** | | |
|---|---|---|---|---|
| | | hSERT | hNET | hDAT |
| | | 0.37 | 594 | 155 |
| Nicotinic acetylcholine | 5.050 | x13.648 | x8.5 | x32 |
| 5-HT3 | 1.040 | x2.810 | x1.7 | x6.7 |

### Conclusion

Compound (I) is very selective for the intended receptors SERT, NET, and DAT, while only binding to a few other receptors with substantially lower inhibition.

### Example 4. Neurochemical mechanism of action in vivo - microdialysis

The purpose of the present study was to examine, by in vivo microdialysis technique, the effects after administration of compound (I) on brain levels of serotonin, noradrenalin and dopamine within the ventral hippocampus (Hipp), prefrontal cortex (PFC), striatum (STR) and nucleus accumbens (N.Acc) of anaesthetized rats and mice. Compound (I) represents dual acting serotonin/dopamine reuptake inhibitors and was tested in comparison to citalopram a selective serotonin uptake inhibitor. Compound (I) was tested following subcutaneous injections at dose levels between 0.25-30 mg/kg. Compound (I) produced a dose dependent increase of serotonin (5-HT) in PFC shown as an area under curve (AUC ₀₋₁₆₀ₘᵢₙ) in Table 6 below: the 0.25 mg/kg dose induced an 190±15 % increase, and almost maximal increase at 1 mg/kg up to an (AUC ₀₋₁₆₀ₘᵢₙ) 252±48%, since the 10 mg/kg dose induced no further increase i.e. 263±47%. Pretreatment with the 5-HT_{1A} autoreceptor antagonist WAY-100635 induced a further minor increase of the compound (I) (0.25 mg/kg) induced increase in the PFC level of 5-HT.

Compound (I) at 1 mg/kg induced a marginal 132±9% increase and at 10 mg/kg a further increase of 211±40 % dopamine (DA) levels in PFC.

Compound (I) at 10 mg/kg produced only a moderate 135±4 % increase of noradrenaline (NA) in PFC (AUC ₀₋₁₆₀ₘᵢₙ). Compound (I) at 10 - 30 mg/kg produced a dose dependent and marked increase of DA in the N.Acc. to levels of 192±42 and 604±88%, respectively. Compound (I) tested at 3 and 10 mg/kg s.c. produced only marginal effects on DA in the striatum (AUC ₀₋₁₆₀ₘᵢₙ) 137±21 and 134±43 %, respectively.

When tested in the Hipp, Compound (I) at 3 mg/kg s.c. markedly increased the 5-HT level (AUC ₀₋₁₆₀ₘᵢₙ) 433±53 %, but less efficiently the levels of noradrenaline (AUC ₀₋₁₆₀ₘᵢₙ) 138±13 % and dopamine (AUC₀₋₁₆₀ₘᵢₙ) 142±36 %.

Comparative data from similar tests was obtained with citalopram 5 mg/kg i.p. The results showed that the effect of citalopram administered at 5 mg/kg induced almost identical effects as compound (I) at 3 mg/kg measured on 5-HT levels in rat Hipp. In the PFC both compound (I) and citalopram increased the 5-HT level but only compound (I) induced effects on DA and minor effects on the NA levels.

**Table 6. Microdialysis profile of compound (I). ND = Not Detectable.**

| **Brain region & subcutaneous doses** | **5-HT (AUC ₀₋₁₆₀ₘᵢₙ)** | **NA (AUC ₀₋₁₆₀ₘᵢₙ)** | **DA (AUC ₀₋₁₆₀ₘᵢₙ)** |
|---|---|---|---|
| Pre-frontal cortex (0.25 mg/kg) | 190±15 | ND | ND |
| Pre-frontal cortex WAY100365 (0.1 mg/kg) + compound (I) (0.25 mg/kg) | 253±32 | ND | 55±2 |
| Pre-frontal cortex (1 mg/kg) | 252±48 | ND | 132±9 |
| Pre-frontal cortex (10 mg/kg) | 263±47 | 135±4 | 211±40 |
| Hippocampus (3 mg/kg) | 433±53 | 138±13 | 142±36 |
| N.Acc (10 mg/kg) | 151±17 | ND | 192±42 |
| N.Acc (30 mg/kg) | ND | ND | 604±88 |
| Striatum (3 mg/kg) | 138±31 | ND | 137±21 |
| Striatum (10 mg/kg) | 170±12 | ND | 134±43 |

### Conclusion

The overall results showed that compound (I) represent a potent in vivo serotonin and dopamine reuptake inhibitor with a well balanced increase in the 5-HT and DA level in PFC together with only a minor influence on the NA levels.

### Example 5: Neurochemical mechanism of action in vivo - the MPTP model of DAT inhibition

Further the MPTP depletion model strongly underlines that compound (I) in-vivo has potency as an inhibitor of the dopamine transporter (DAT) in the C57 mouse striatal tissue producing approximately 90 % protection at a dose of 15 mg/kg and a marginal further effect at 30 mg/kg (Table 7). In this model compound (I) induced protection against the dopamine depletion induced by the entry of the MPTP metabolite MPP+ into the dopamine terminals of the mouse C57 striatal tissue.

**Table 7. Compound (I) dosed prior to MPTP at 7.5, 15 and 30 mg/kg p.o.**

| **Treatment** | **Dopamine Mean±SEM** | **DOPAC Mean±SEM** | **HVA Mean±SEM** |
|---|---|---|---|
| Placebo | 13.7 ± 2.9 | 1 ± 0.2 | 1.1 ± 0.2 |
| MPTP 25mg/kg s.c. | 4.2 ± 0.6 | 1 ± 0.2 | 0.7 ± 0.1 |
| Compound (I) 7.5 mg/kg p.o. 0 min + MPTP 25 mg/kg s.c. 60 min. | 6,3 ± 1,1 | 1 ± 0.2 | 0.7 ± 0.1 |
| Compound (I) 15 mg/kg p.o. 0 min + MPTP 25 mg/kg s.c. 60 min. | 12.5 ± 0.7 | 1,7 ± 0.1 | 1 ± 0.1 |
| Compound (I) 30 mg/kg p.o. 0 min + MPTP 25 mg/kg s.c. 60 min. | 14.7 ± 1.2 | 2.1 ± 0.2 | 1.2 ± 0.1 |

### Conclusion

Compound (I) has potency as an inhibitor of the dopamine transporter (DAT) in the C57 mouse striatal tissue.

### Example 6: PD/PK studies in mice and rats

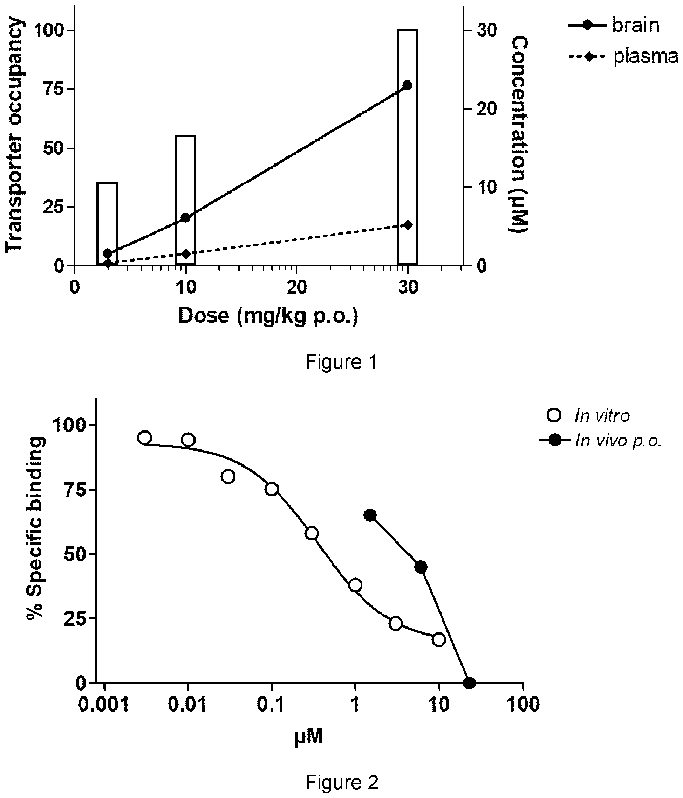

The relationship between brain concentrations of compound (I) and transporter occupancy was investigated in *in vivo* ³H-WIN 35.428 binding in mice. A relationship between increasing plasma and brain concentrations with each increase in dose level, and a corresponding increase in CNS *in vivo* DA transporter occupancy 90 min post administration. In both cases there was a close correlation between plasma and brain concentrations and in vivo transporter occupancy in brain, the brain to plasma ratio was approximately 4 at 3, 10 and 30 mg/kg (Figure 1). Compound (I) concentration-dependently inhibits *in vitro* and *in vivo* WIN binding in striatum (Figure 2). The inhibition of *in vivo* WIN binding was measured 90 min after p.o. administration of the compound.

### Example 7: In vivo transporter occupancy

The *in vivo* receptor occupancy (RO%) of compound (I) was calculated at the MED values (DOSE) in mFST and in the mouse motility test using the formula RO% (estimated) = DOSE · 100 / (ED₅₀ + DOSE). The calculations were based on ED₅₀ values in *ex vivo* uptake of 5-HT and NA and WIN *in vivo* binding. The results are shown in Table 8.

**Table 8. Estimated receptor occupancy level. 1: ED₅₀ = ED₅₀ in ex vivo 5-HT uptake in mice = 1.9 mg/kg p.o. 2) ED₅₀ = ED₅₀ in ex vivo NA uptake in mice = >50 mg/kg p.o. 3) ED₅₀ = ED₅₀ in WIN in vivo binding in mice = 9.9 mg/kg p.o.**

| **Model** | **DOSE (mg/kg)** | **5-HT RO%¹** | **NA RO%²** | **DA RO%³** |
|---|---|---|---|---|
| mFST (NMRI mice) | 1 | 34 | <1.9 | 9 |
| mMotility | 10 | 84 | <17 | 50 |

### Example 8: Abuse liability

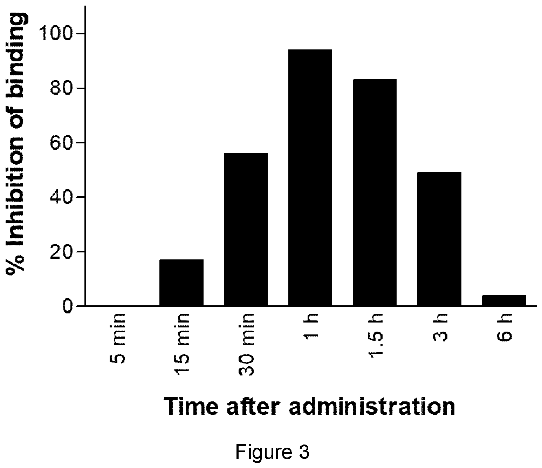

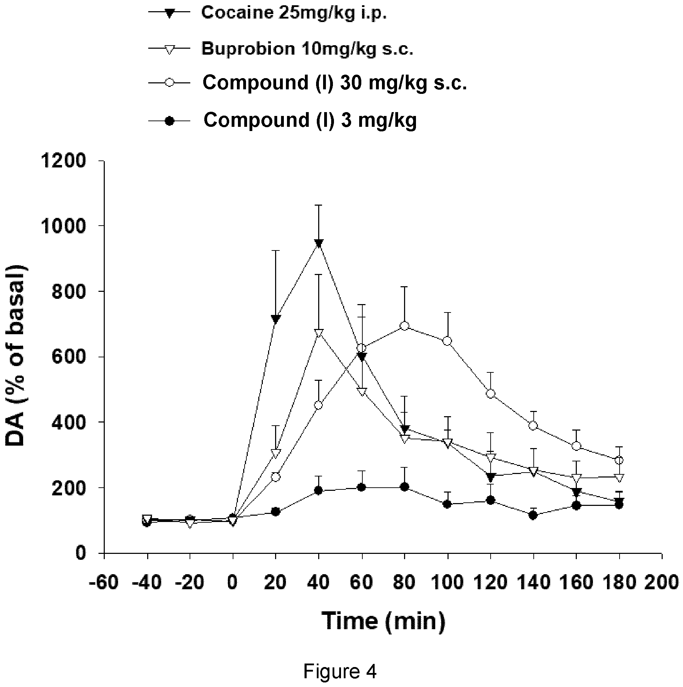

In an in vivo binding time course study in mice dosed 20 mg/kg p.o., the inhibition of WIN binding increased over time demonstrating a slow onset of DA transporter blockade in vivo (Figure 3). Compound (I) 30 mg/kg s.c. in rats shows slower kinetic pattern in microdialysis (nucleus accumbens) compared to bupropion 10 mg/kg s.c. and cocaine 25 mg/kg i.p. In vivo binding data are shown in Figure 4. It is known that the critical factor for psychomimetic side effects of a dopaminergic drug is the speed of inhibition of the DA transporter rather than the increase in DA release per se. This slower kinetic pattern could indicate a lower risk of abuse potential.

### Example 9: In vivo pharmacological activity - in vivo efficacy on depression, and mechanism thereof

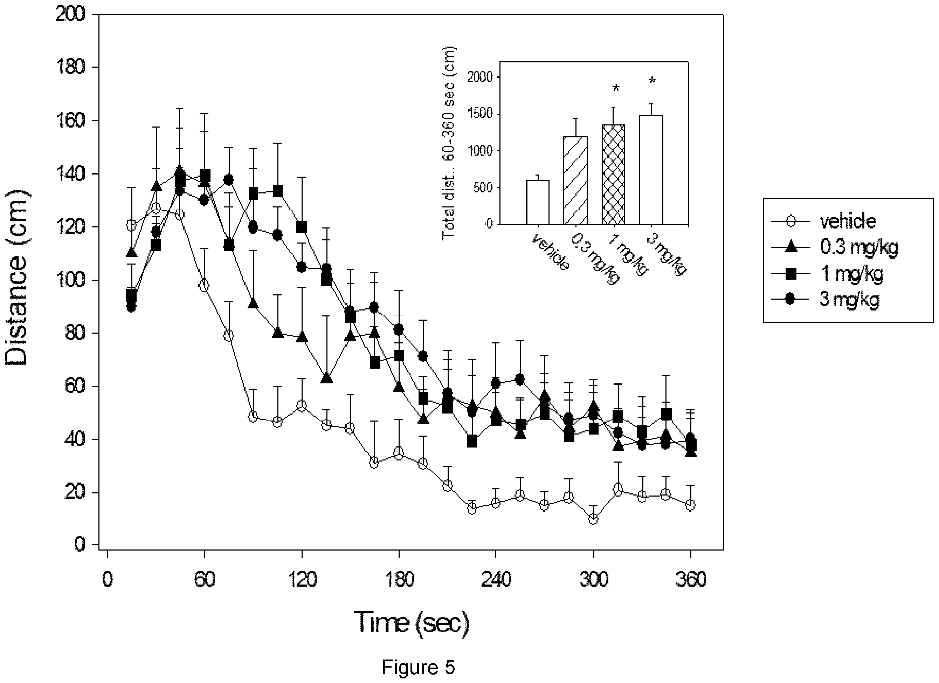

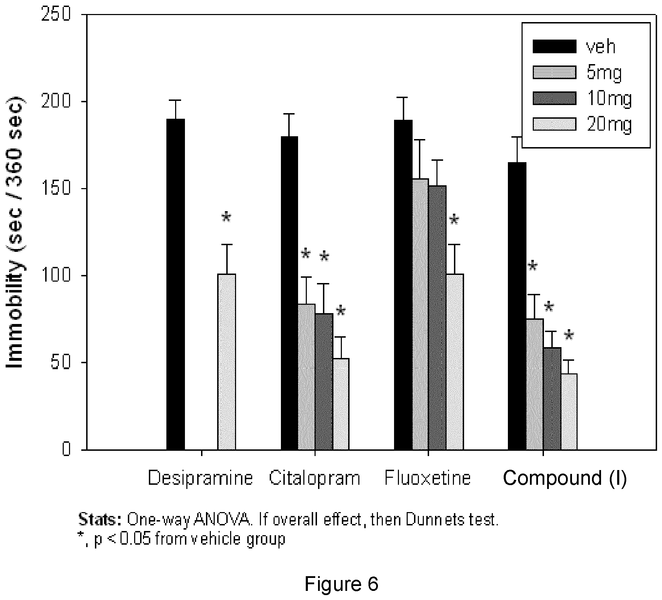

Compound (I) was tested in two of the most widely used behavioural screens for predicting antidepressant effects in the clinic: the mouse forced swim test (mFST) and mouse tail suspension test (mTST). These paradigms display a high degree of pharmacological validity as evidenced by their sensitivity to major classes of antidepressant treatments: tricyclic compounds, monoamine oxidase inhibitors, atypical antidepressants, selective serotonin reuptake inhibitors (SSRIs), serotonin-noradrenaline reuptake inhibitors (SNRls) and electroconvulsive shock. As outlined in the Table 9 and Figure 5 and Figure 6, compound (I) significantly increased swim distance and reduced immobility time in the mFST and mTST, respectively, when compared to vehicle-treated mice. These effects are indicative of potential antidepressant-like activity. In the mFST, the effects of compound (I) were more potent than those obtained following treatment with reference compounds (mFST: compound (I) > citalopram > duloxetine). In the mTST, the effects of compound (I) were equipotent or more potent than those obtained following treatment with reference compounds (compound (I) = citalopram > fluoxetine).

**Table 9. Summary of animal models of depression.**

| **Compound** | **Model** | **Effect/Comments** | **MED** |
|---|---|---|---|
| Compound (I) | mFST (NMRI) | 0.3, 1, 3 mg/kg (p.o. 60 min) | 1 mg/kg |
| | mTST (C57) | 5, 10, 20 mg/kg (i.p. 30 min) | < 5 mg/kg |
| Duloxetine | mFST (NMRI) | 3, 10, 30 mg/kg (p.o. 60 min) | 30 mg/kg |
| | mTST (C57) | 5, 10, 20 mg/kg (i.p. 30 min) | 10 mg/kg |
| Citalopram | mFST (NMRI) | 3, 10, 30 mg/kg (p.o. 60 min) | 10 mg/kg |
| | mTST (C57) | 5, 10, 20 mg/kg (i.p. 30 min) | < 5 mg/kg |

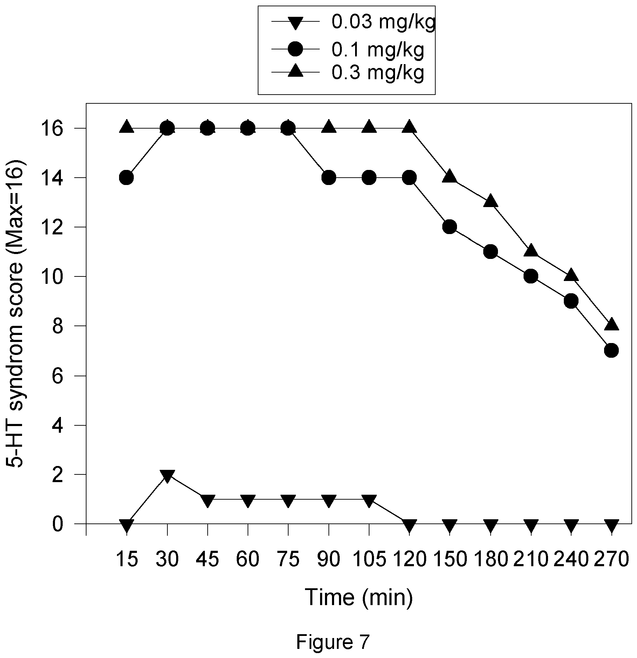

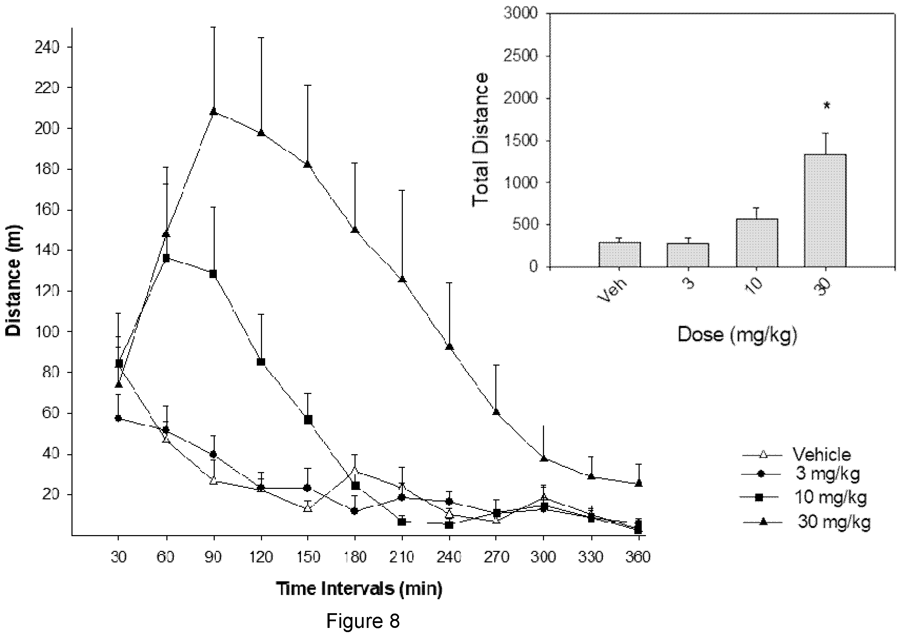

Compound (I) was tested in a classical test often used to measure the ability of a compound to block the reuptake of 5-HT *in vivo:* nialamide-induced 5-HT syndrome (m5-HT). In addition, compound (I) was tested in a locomotor activity paradigm (mLA) as a measure of its ability to block the reuptake of DA and therefore induce motility. It is generally accepted that >60 % DA transporter blockade is needed to induce increased motility in this test. As outlined in the Table 10 and Figure 7 and Figure 8, compound (I) potently enhanced 5-HT syndrome-like behaviour in nialamide pre-treated mice, activity indicative of in vivo 5-HT reuptake inhibition. The effects of compound (I) were 100 times more potent than those obtained with the reference compounds tested. In addition, compound (I) increased locomotor activity at 10 mg/kg (2 h test) and 30 mg/kg (6 h test) indicating DA reuptake inhibitor properties, whereas duloxetine and citalopram were without effect. The effects of compound (I) on motility were of slow onset and long duration.

**Table 10. Summary in mechanistic models.**

| **Compound** | **Model** | **Effect/Comments** | **MED** |
|---|---|---|---|
| Compound (I) | m5-HT | 0.03, 0.1, 0.3 mg/kg (p.o. 0 min) | 0.1 mg/kg |
| | mLA | 3, 10, 30 mg/kg (p.o. 0 min) | 10 mg/kg |
| Duloxetine | m5-HT | 1, 3, 10 mg/kg (p.o. 0 min) | 10 mg/kg |
| | mLA | 10, 30, 60 mg/kg (p.o. 0 min) | >60 mg/kg |
| Citalopram | m5-HT | 1, 3, 10 mg/kg (p.o. 0 min) | 10 mg/kg |
| | mLA | 60 mg/kg (p.o. 0 min) | >60 mg/kg |

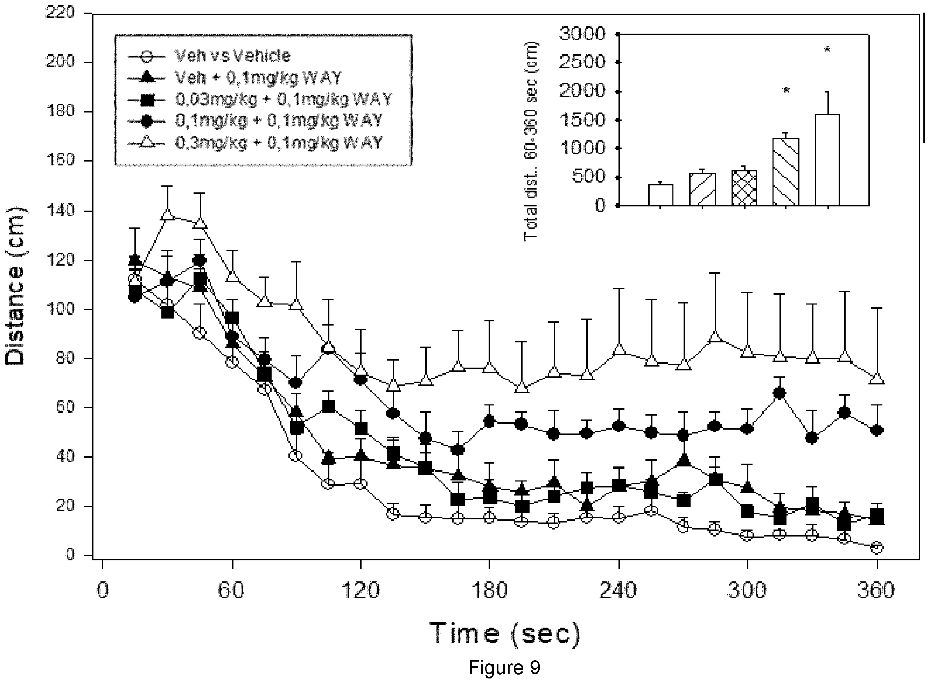

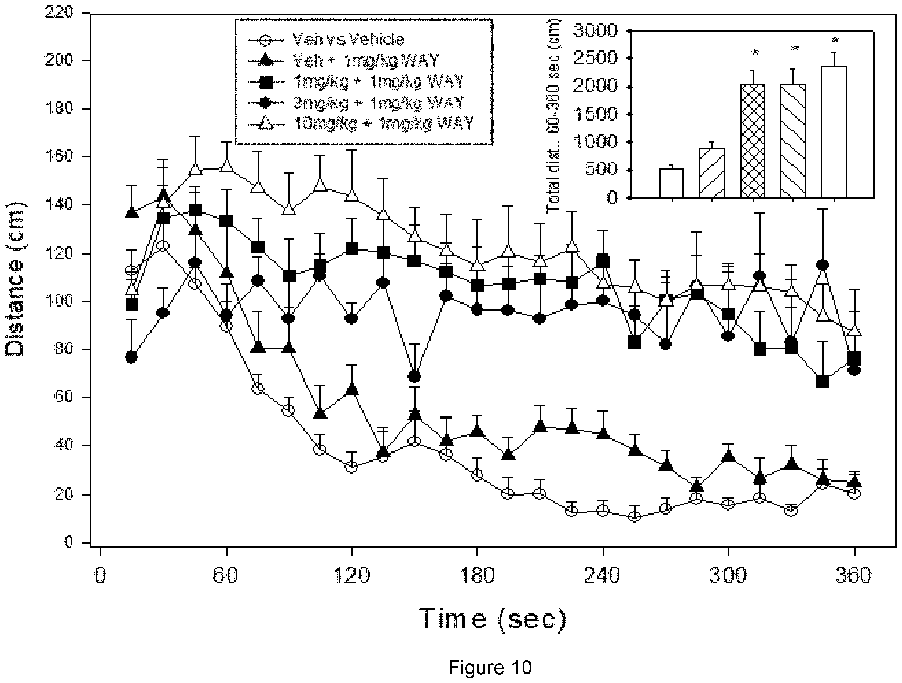

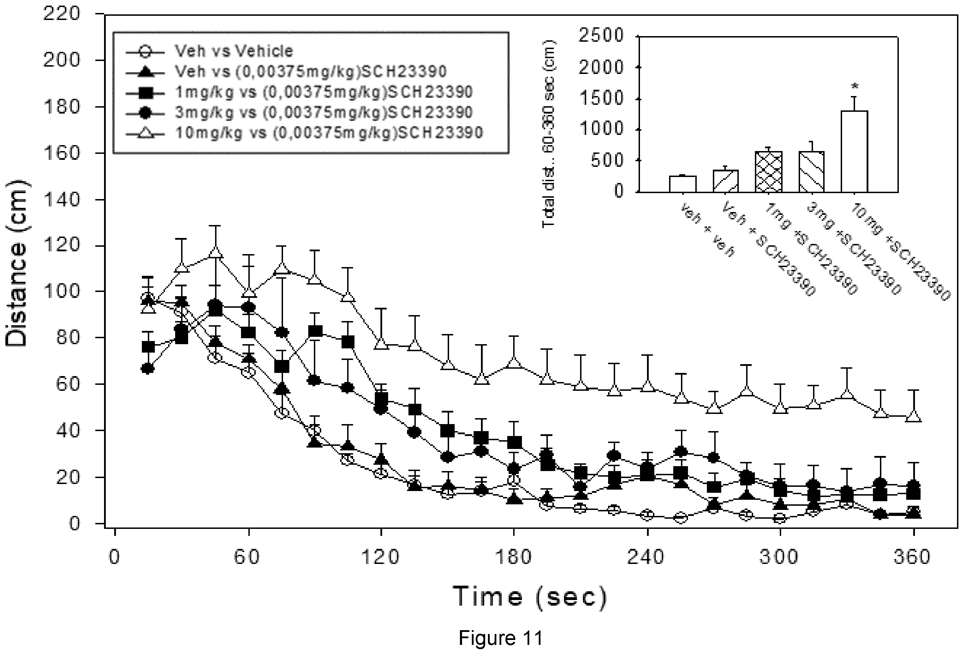

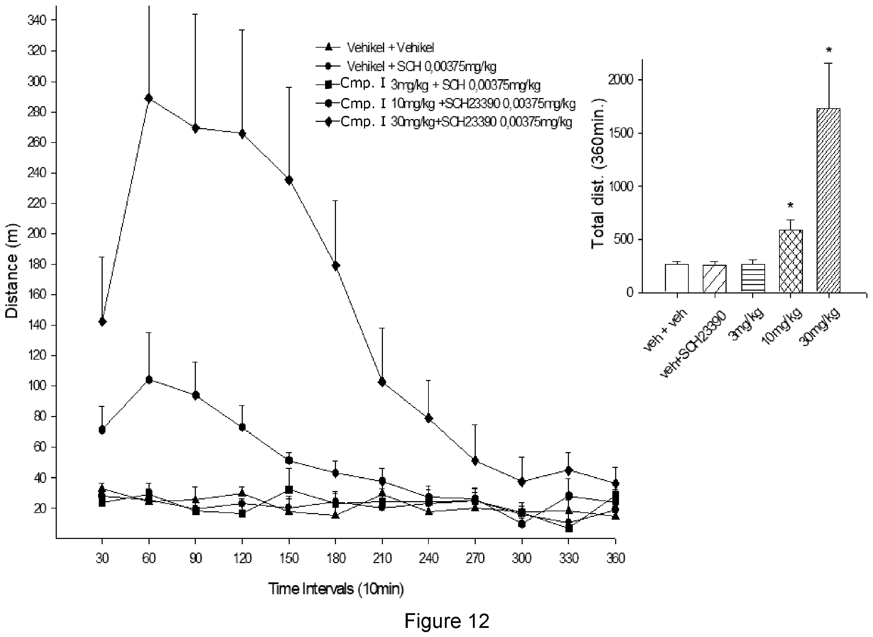

In order to further characterize the role played by 5-HT and DA-ergic neurotransmission in the antidepressant-like effects of compound (I), the potential involvement of 5-HT1A and D1 receptors was investigated in the mFST. Sub-active/active doses of the compound were tested in combination with WAY1006351 (5-HT1A receptor antagonist) and SCH23390 (D1 receptor antagonist), respectively. As shown in the Table 11 and Figure 9 and Figure 10, combination of a low dose of WAY100635 (most likely blocking presynaptic 5-HT1A receptors) with normally sub-active doses of compound (I) resulted in significant antidepressant-like activity in the mFST (MED: 0.1 mg/kg, 10-fold leftward shift in dose-response), implicating increased 5-HT function in the mechanism of action of compound (I). These data are also in good agreement with microdialysis studies showing augmented 5-HT in PFC when compound (I) is combined with WAY100635. In contrast, combination of a higher dose of WAY100635 (most likely blocking both pre- and postsynaptic 5-HT_{1A} receptors) with active doses of compound (I) did not influence the activity of the compound (MED: 1 mg/kg). This result suggests that DA may already be involved at doses as low as 1 mg/kg. As shown in Figure 11 and Figure 12, the antidepressant-like activity of compound (I) was attenuated when active doses were combined with a non-sedative dose of SCH23390 in the mFST (MED: 10 mg/kg, 10-fold rightward shift in dose-response), suggesting increased DA function at active doses (D1 receptor mediated). This hypothesis is in good agreement with microdialysis studies showing enhanced DA release in PFC at similar doses, and may explain the data obtained using the higher dose of WAY100635. Interestingly, combination of compound (I) with SCH23390 did not affect the overall locomotor activity profile of compound (I), suggesting that the antidepressant-like activity of the compound is most likely mediated via PFC and possibly N.acc 5-HT/DA interactions, and not striatum.

**Table 11. Summary of combination studies with WAY100635 and SCH23390.**

| **Model** | **Effect/Comments** | **MED** |
|---|---|---|
| mFST | Compound (I) (0.03, 0.1, 0.3 mg/kg, p.o. -60 min) + WAY100635 (0.1 mg/kg, s.c. -60 min) | 0.1 mg/kg |
| mFST | Compound (I) (1, 3, 10 mg/kg, p.o. -60 min) + WAY100635 (1 mg/kg, s.c. -60 min) | 1 mg/kg |
| mFST | Compound (I) (1, 3, 10 mg/kg, p.o. -60 min) + SCH23390 (0.00375 mg/kg, s.c. -60 min) | 10 mg/kg |
| mLA | Compound (I) (1, 3, 10 mg/kg, p.o., -60 min) + SCH23390 (0.00375 mg/kg, s.c., -60 min) | 10 mg/kg |

### Conclusion

Compound (I) exhibited significant activity in the 5-HT syndrome (MED: 0.1 mg/kg) assay commensurate with being a 5-HT reuptake inhibitor. The compound also induced significant increases in locomotor activity (corresponding to >60% DA transporter inhibition) at higher doses (10 and 30 mg/kg, p.o) indicating potent DA reuptake inhibitor properties. Furthermore, when tested in simple efficacy assays (mFST, mTST), compound (I) displayed robust antidepressant properties. Efficacy in these assays appeared to involve both 5-HT and DA mechanisms within the same dose range, as evidenced by combination studies employing selective antagonists. In conclusion, it appears that compound (I) represents a novel 5-HT/DA reuptake inhibitor possessing potential antidepressant-like properties.

### Comparative Example 10: In vivo efficacy on anxiety

Compound (I) was tested in three widely used behavioural screens for predicting anxiolytic effects in the clinic: the mouse marble burying test (mMB), mouse zero maze (mZM) and mouse stress-induced hyperthermia test (mSIH).

**Table 12. Summary in animal models of anxiety**

| **Compound** | **Model** | **Effect/Comments** | **MED** |
|---|---|---|---|
| Compound I | Marble burying | 0.1, 0.3, 1 mg/kg (p.o., -60 min) | 0.1 mg/kg |
| | mZero maze | 1, 3, 10 mg/kg (p.o., -60 min) | > 10 mg/kg |
| | mZero maze | 0.3, 1, 3 mg/kg (p.o., 7 days OD) | <0.3 mg/kg |
| | mSIH | 0.3, 1, 3 mg/kg (p.o., -60 min) | 0.3 mg/kg |
| | | | |
| Duloxetine | Marble burying | 3, 10, 30 mg/kg (p.o., -60 min) | 10 mg/kg |
| | mZero maze | Acute | ND |
| | mZero maze | 10 mg/kg (p.o., 7 days OD) | < 10 mg/kg |
| | mSIH | 3, 10, 30 mg/kg (p.o., -60 min) | > 30 mg/kg |

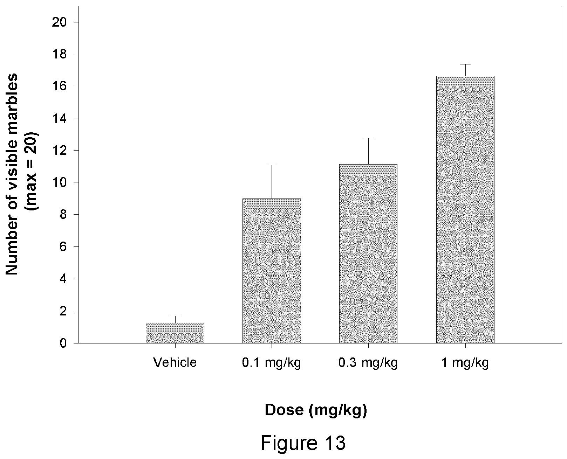

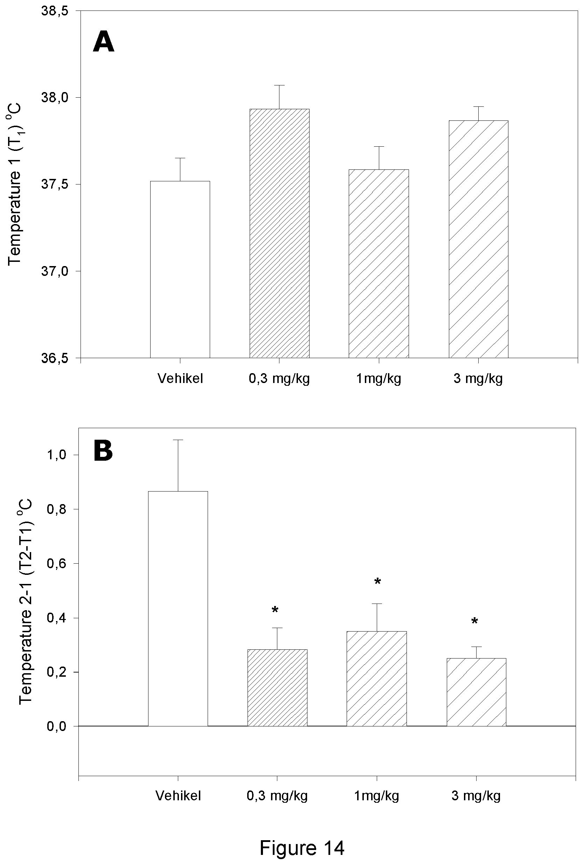

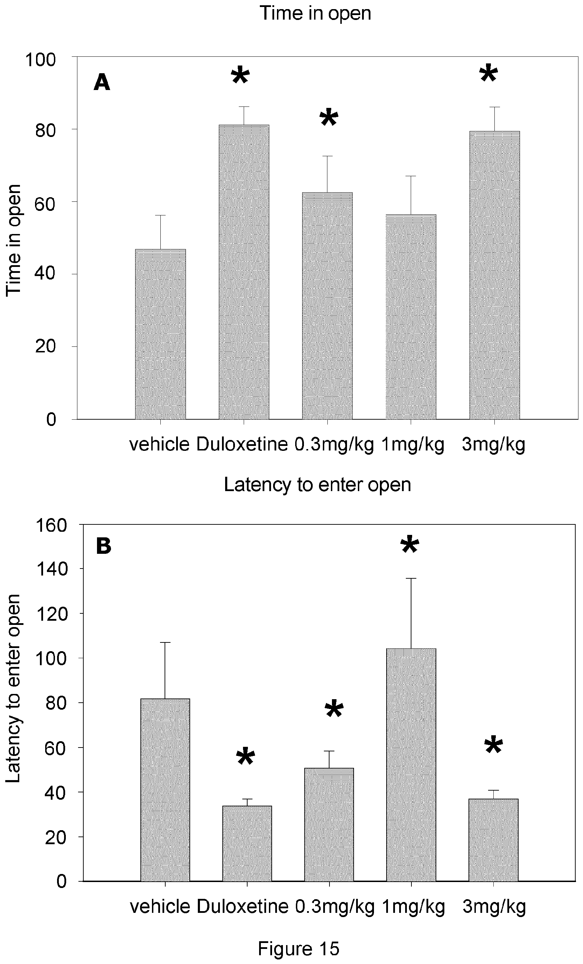

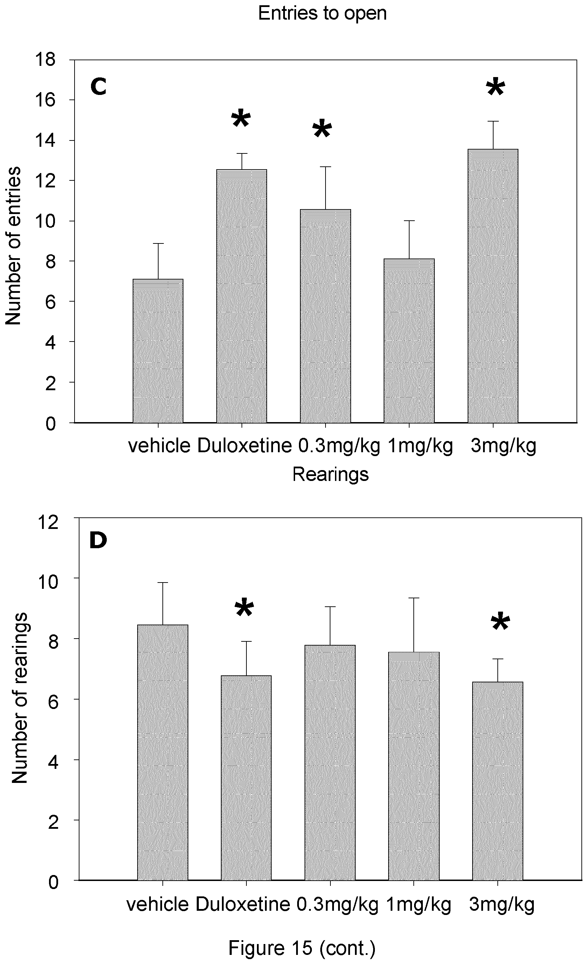

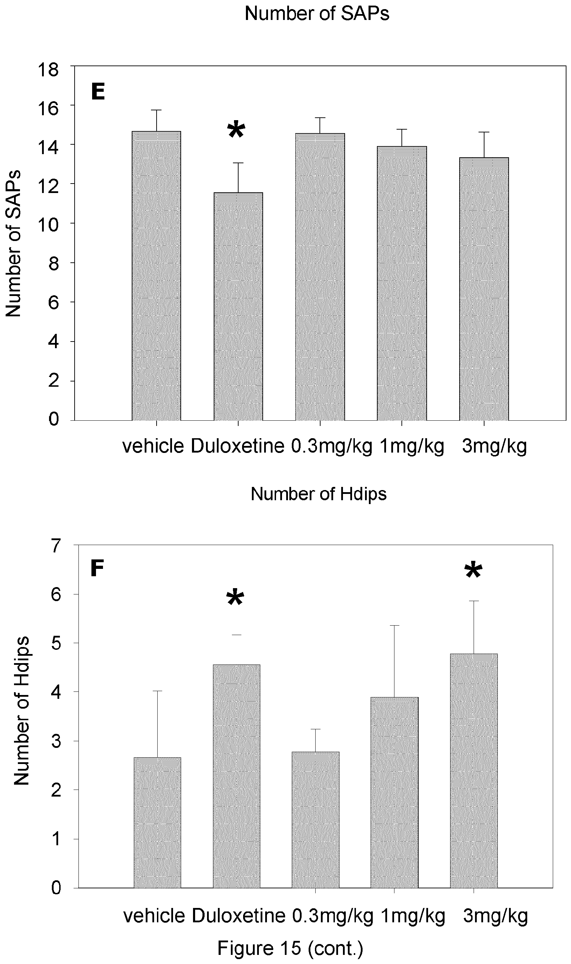

As outlined in table 12 and Figs. 13, 14 and 15, Compound I significantly decreased burying behaviour in the mMB test and increased time spent in open in the mZM test after repeated treatment, as did duloxetine. The anxiolytic-like effects of Compound I were more potent than duloxetine in the mMB test and equipotent in the mZM test. Most interestingly, Compound I attenuated stress-induced hyperthermia in the mSIH test, effects that could not be obtained following duloxetine treatment. In general, second and third generation antidepressants are inactive in this test, and thus highlights the unique properties of Compound I in an animal model of anticipatory anxiety.

### Example 11: Preclinical safety pharmacology - HERG channel testing

A HEK293 cell line stably expressing Kv11.3 for hERG and KCNE1 (minK) was established. The effect of compound (I) on the amount of current carried by hERG channels was assessed in whole-cell patch clamp experiments. The channels were activated by a voltage protocol designed to simulate a human cardiac action potential. At 10 µM, compound (I) blocked the current with an estimated Ki of >10 µM.

### Example 12: Preclinical safety pharmacology - cardiovascular safety in anaesthetised dogs

No consistent changes in arterial blood pressure (mean, systolic and diastolic) were observed following the administration of 1, 3, 10 and 30 mg/kg i.v. compound (I) to α-chlorulose anaesthetized dogs, with the exception of an apparent dose-dependent decrease following 10 and 30 mg/kg compound (I). Dose-dependent increases in heart rate were observed following the administration of 1, 3, 10 and 30 mg/kg compound (I), which was associated with concurrent dose-dependent decreases in the RR interval.

The PR interval and QRS duration were relatively unaffected by the administration of 1, 3, 10 and 30 mg/kg compound (I), with the exception of the PR interval were was a potential shortening of this interval following the administration of 30 mg/kg compound (I). No consistent changes in the QT interval were observed following the administration of 1, 3, 10 and 30 mg/kg compound (I). When corrected for changes in heart rate / RR interval to produce the QTcF and QTcV intervals a clear dose-dependent increase was observed, This was also shown following periods when the heart was paced at either 90 or 110 bpm (Figure 16 and Figure 17), but as indicated in Table 13 this occurred at plasma concentrations between 6500 - 15,000 ng/ml.

**Table 13: Total plasma concentrations of compound (I).**

| | | | | |
|---|---|---|---|---|
| Bolus i.v. dose | 1.0 mg/kg | 3.0 mg/kg | 10.0 mg/kg | 30.0 mg/kg |
| Average conc. of compound (I) at 5 min (max) | 631 ng/ml | 2120 ng/ml | 6560 ng/ml | 15000 ng/ml |

### Example 13: Bioavailability in mouse and rat

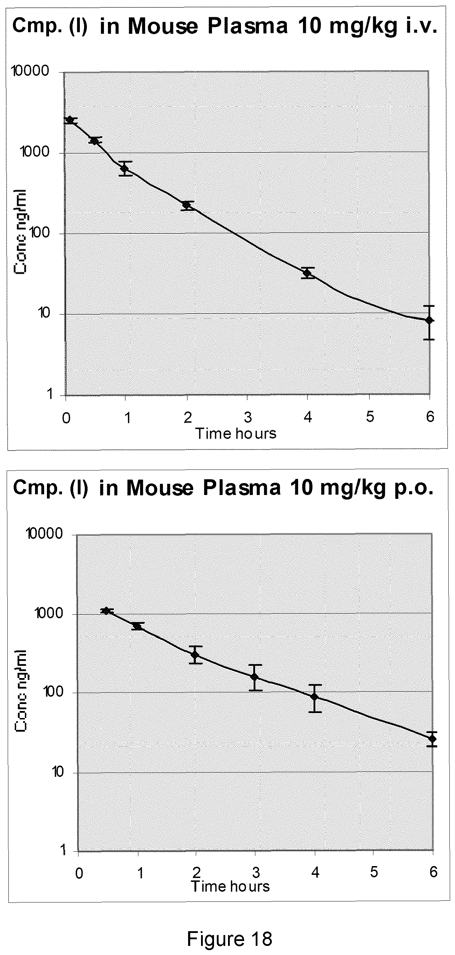

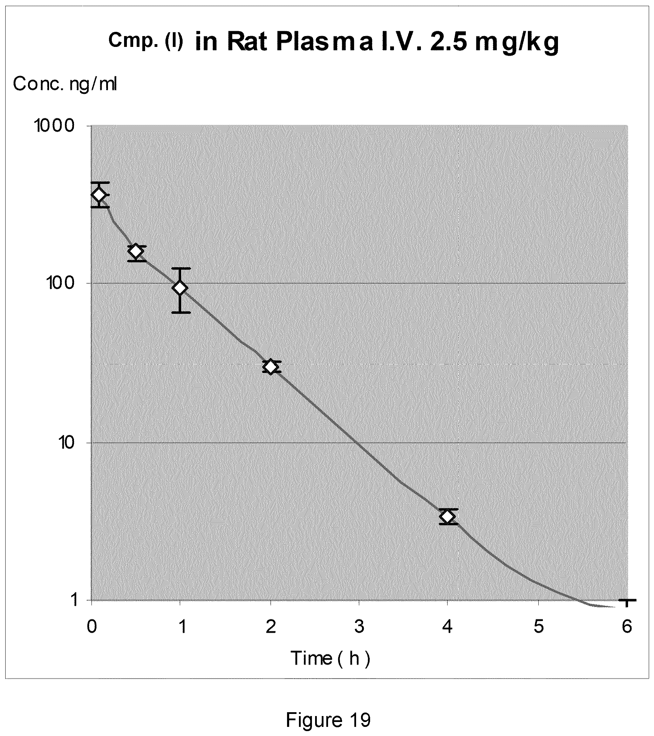

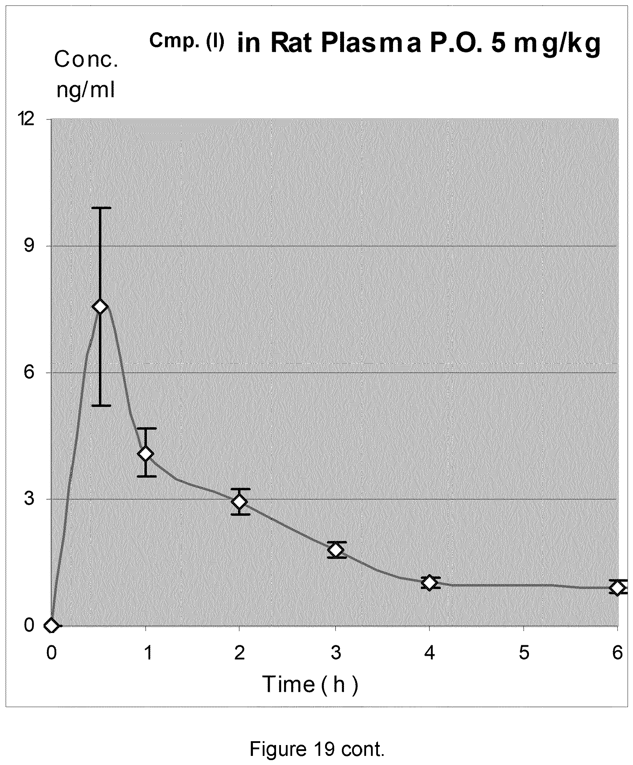

Bioavailability studies was performed in female NMRI mice and male Wistar rats (N=4). Plasma samples were withdrawn up to 24 h. These studies show that the bioavailability is best in mouse (74%). The bioavailability in rat is 3%, why the following studies performed in rats is using s.c. dosing. Compound (I) is absorbed quickly. Plasma PK profiles in mice show 1st order elimination kinetics (See Figure 18 and Figure 19). The T½ is relatively fast after i.v. dosing; 0.6 and 0.8 h in mouse and rat respectively. Table 14 shows a summary of PK parameters for bioavailability studies in the rodents.

**Table 14. PK parameters from bioavailability studies in mouse and rat. *: Rats could not tolerate 10 mg/kg i.v. because of exaggerated dopaminergic effects. NA: Not applicable, plasma concentrations close to limit of quantification.**

| **Compound (I)** | **Mouse** | **Rat** |
|---|---|---|
| *p.o.* dose (clear solution) | 10 mg/kg | 5 mg/kg |
| *i.v.* dose (clear solution) | 10 mg/kg | 2.5 mg/kg* |
| Bioavailability | 74% | 3% |
| T½ *i.v.* | 0.8 h | 0.6 h |
| T½ *p.o.* | 1.1 h | NA |
| Vd | 3.5 L/kg | 5.6 L/kg |
| Clearance | 4.3 L/h/kg | 8.2 L/h/kg |
| AUC_{(0-∞)} *p.o*. | 1715 ng/ml·h | 17 ng/ml·h |
| AUC_{(0-∞)} *i.v.* | 2308 ng/ml·h | 306 ng/ml·h |
| Tₘₐₓ *p.o*. | 0.5 h | 0.5 h |
| Cₘₐₓ *p.o*. | 1086 ng/ml | 8 ng/ml |
| Vehicle | 0.9% NaCl, pH 5 | 5.5% glucose |

### Example 14: Bioavailability in dog

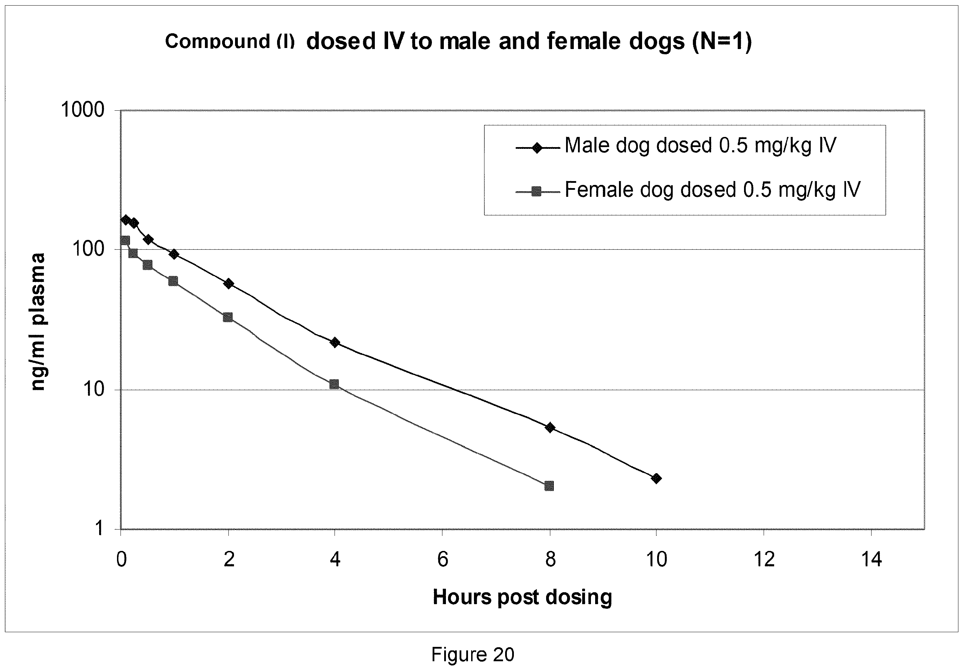

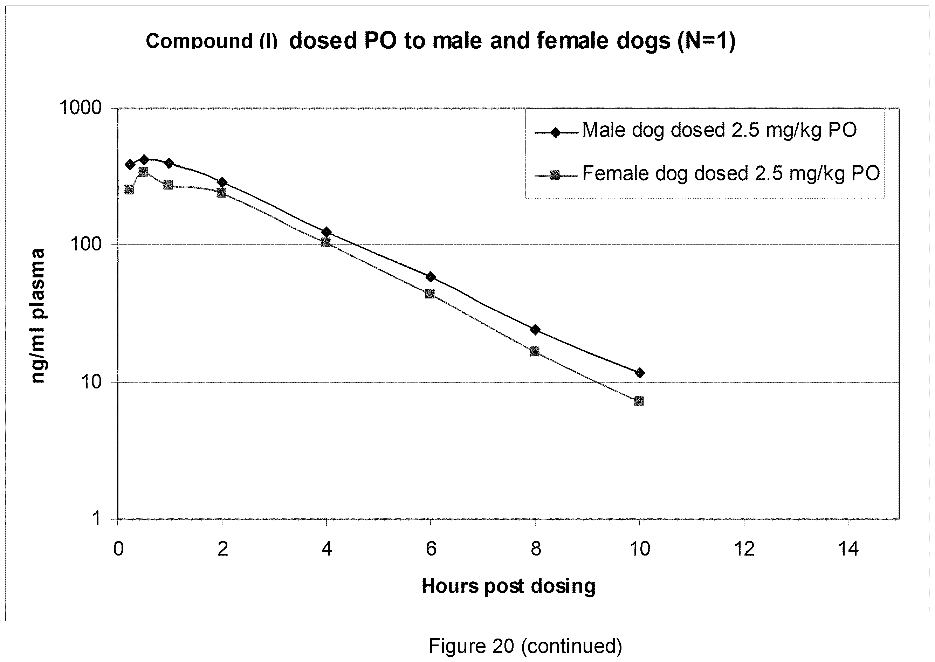

A bioavailability study was also conducted in dogs. One female and one male dog were dosed 2.5 mg/kg p.o. and 0.5 mg/kg i.v. After the dose 2.5 mg/kg some CNS effects were observed in both the male and the female dog. The i.v. dose, which was given 7 days later, was chosen as 0.5 mg/kg in order to prevent observable CNS effects. The difference between the p.o. and the i.v. dose might overestimate the bioavailability to a certain extent. The bioavailability was estimated to 84% in male dog and 114% in female. Plasma elimination kinetics showed 1st order kinetics after i.v. dosing, Figure 20. T½ is in the same range in the two dogs and after both p.o. and i.v. administration (1.4-1.7 h).

**Table 15: PK parameters from bioavailability studies in dog.**

| **Compound (I)** | **Male beagle dog** | **Female beagle dog** |
|---|---|---|
| *p.o.* dose (clear solution) | 2.5 mg/kg | 2.5 mg/kg |
| *i.v.* dose (clear solution) | 0.5 mg/kg | 0.5 mg/kg |
| Bioavailability | 84% | 114% |
| T½ *i.v.* | 1.6h | 1.4 h |
| T½ *p.o.* | 1.7 h | 1.6 h |
| Vd | 3.4 L/kg | 5.0 L/kg |
| Clearance | 1.5 L/h/kg | 2.6 L/h/kg |
| AUC_{(0-∞)} *p.o.* | 1437 ng/ml·h | 1106 ng/ml·h |
| AUC_{(0-∞)} *i.v.* | 343 ng/ml·h | 194 ng/ml·h |
| Tₘₐₓ *p.o.* | 0.5 h | 0.5 h |
| Cₘₐₓ *p.o*. | 418 ng/ml | 339 ng/ml |
| Vehicle | 5% glucose | 5% glucose |

### Example 15: Linearity studies in mouse and rat after single dose

PK data from dosing rats s.c. in relatively low doses, show very nice linear kinetics, when depicted as AUC versus dose. However, PK data from dosing mice in relatively high doses p.o., show a tendency to sub-linear kinetics.

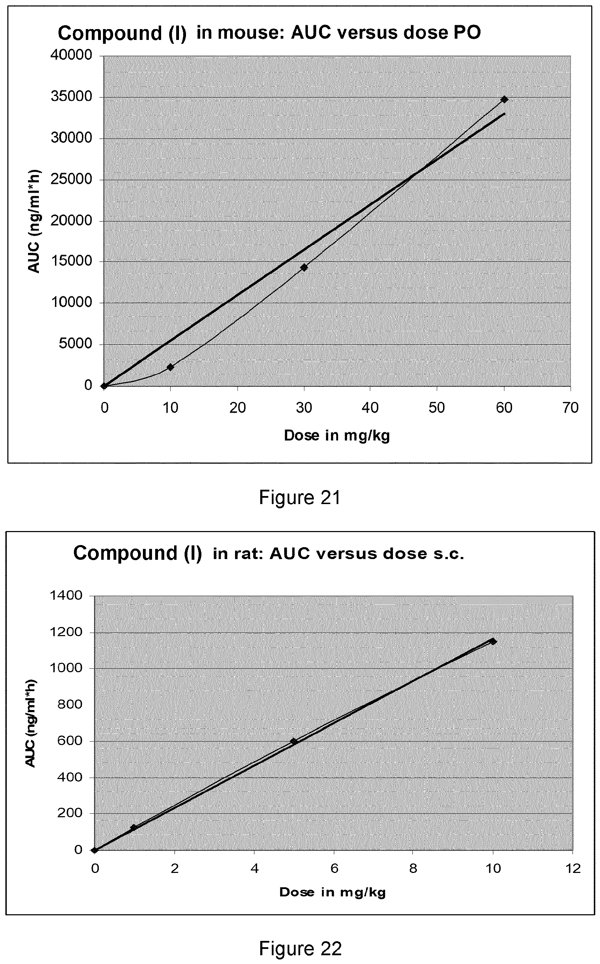

Compound (I) was dosed to mouse at 30 and 60 mg/kg p.o. Plasma samples were withdrawn up to 24 h. The results are summarised in Table 16 and Figure 21.

**Table 16. Compound (I) dosed to mouse 10, 30, and 60 mg/kg p.o. as a single dose. *: compared to 10 mg/kg i.v.**

| **Compound (I)** | **Mouse (female NMRI)** | | |
|---|---|---|---|
| *p.o.* doses (clear solution) | **10 mg/kg *p.o.*** | **30 mg/kg *p.o.*** | **60 mg/kg *p.o.*** |
| Vehicle | 0.9% NaCl | 5% Tween 80 | 5% Tween 80 |
| Tₘₐₓ | 0.5 h | 0.5 h | 0.5 h |
| Cₘₐₓ | 1086 ng/ml | 3081 ng/ml | 4148 ng/ml |
| Bioavailability* | 74% | 206% | 250% |
| T½ | 1.1 h | 5.1 h | 6.1 h |
| AUC_{(0-∞)} | 2308 ng/ml·h | 14273 ng/ml·h | 34685 ng/ml·h |

Compound (I) was dosed to rat at 1, 5 and 10 mg/kg s.c. Plasma samples were withdrawn up to 24 h and analysed. The results are summarised in Table 17 and Figure 22.

**Table 17. Compound (I) dosed to rat 1, 5 and 10 mg/kg s.c. as a single dose. *: compared to 10 mg/kg i.v. dosed in same vehicle.**

| **Compound (I)** | **Rat (male Wistar)** | | |
|---|---|---|---|
| *s.c.* doses (clear solution) | **1 mg/kg *s.c.*** | **5 mg/kg *s.c.*** | **10 mg/kg *s.c.*** |
| Vehicle | 5% Tween 80 | 5% Tween 80 | 5% Tween 80 |
| Tₘₐₓ | 1 h | 1 h | 0.5 h |
| Cₘₐₓ | 69 | 320 | 729 |
| Bioavailability* | 117% | 113% | 108% |
| T½ | 0.67 h | 0.77 h | 0.68 h |
| AUC_{(0-∞)} | 125 ng/ml·h | 604 ng/ml·h | 1151 ng/ml·h |

### Example 16: Linearity after repeat dosing in mouse and rat

Single time point data is available from two 14 days repeat dose studies in mice and rats dosed to both sexes. The three low doses were performed together as one study and the top dose was performed at another occasion as a satellite study.

Mice seem to exhibit a tendency to sub-linear kinetics, whereas the rats show a slight tendency to supra-linear kinetics. This difference might be a result of the inherent difference in the way of administration. It should be noted that the compound in these studies was dosed in 5% glucose (clear solution) and not in Tween 80 as in the single dose studies.

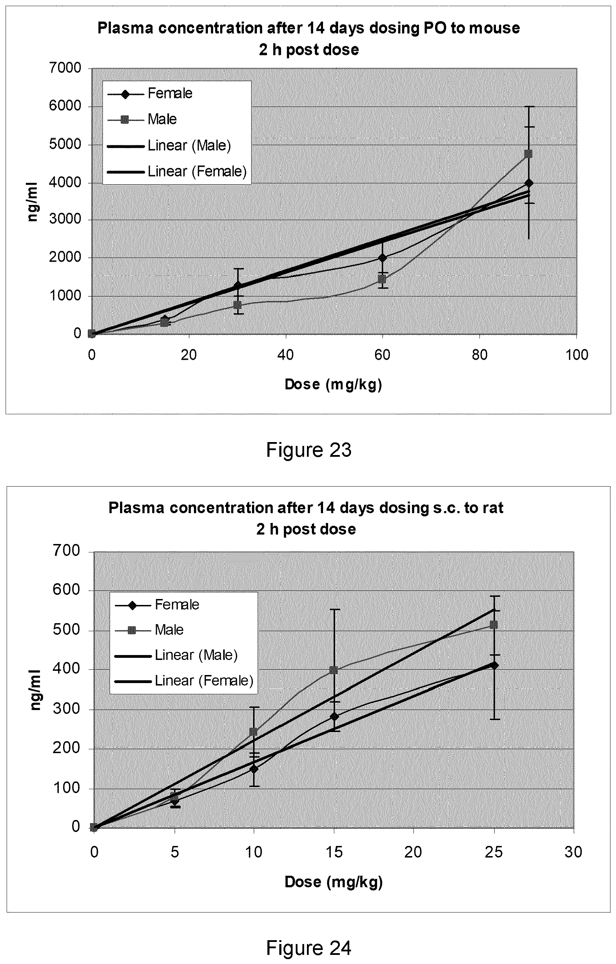

Compound (I) was dosed 15, 30, 60 and 90 mg/kg p.o. for 14 days. Samples were taken 2 h post last dose. Mice seem to exhibit a tendency to sub-linear kinetics after repeat dosing. Especially the male mice show a considerable rise in plasma concentration in the last dose of 90 mg/kg p.o. At doses 15 to 60 mg/kg, the female mice show higher plasma concentrations than male, but at 90 mg/kg the curves cross and the male mice show higher concentrations (Figure 23).

Compound (I) was dosed 5, 10, 15 and 25 mg/kg s.c. for 14 days. Samples were taken 2 h post last dose. Both male and female rats show a slight tendency to supra-linear kinetics after repeat dosing. The male rats exhibit higher concentrations in plasma compared to female. This picture persists from 5 to 25 mg/kg (Figure 24).

### Example 17: Tissue distribution after single dose

Compound (I) was dosed to female NMRI mice 10 mg/kg p.o. Plasma was taken 0.5, 1, 2, 4, 6 and 24 post dose. Brain, liver and lung were taken 1, 4 and 24 hours post dose.

The plasma profile was essentially the same as for the bioavailability study (Table 20), showing the same T½ of 1.1 h. AUC and T½ were calculated for plasma and organs, even though the data are very sparse for the organs. Compound (I) shows a remarkable preference for liver and lung in mouse, whereas the ratio to brain is optimal.

**Table 18. Tissue distribution in mouse after single dose.**

| **Compound (I) dosed to mouse (female NMRI) 10 mg/kg *p.o.*** | | | | |
|---|---|---|---|---|
| Organ | Plasma | Brain | Liver | Lung |
| T½ | 1.1 | 3.0 | 2.5 | 2.2 |
| AUC₍₀₋₂₄ₕ₎ ng/ml·h | 2215 | 9353 | 38508 | 35744 |
| AUC_{organ}/AUCₚₗₐₛₘₐ | NA | 4.2 | 17.4 | 16.1 |

Compound (I) was dosed to male Wistar rats 5 mg/kg s.c. Plasma was taken 0.5, 1, 2, 4, 6 and 24 post dose. Brain, liver and lung were taken 1, 4 and 24 hours post dose.

The plasma profile was again essentially the same as for the single dose study showing the same T½ of 0.8 h. AUC and T½ were calculated for plasma and organs, even though the data are very sparse for the organs.

In contrast to mouse, compound (I) is not found in liver in high concentrations (Table 19). This might be due to the subcutaneous dosing bypassing the liver and/or the rat's much higher capacity to metabolise the compound. The distribution to brain is the same as in the mouse. The distribution to lung is a little higher compared to mouse.

**Table 19. Tissue distribution in rat after single dose**

| **Compound (I) dosed tomale Wistar rats 5 mg/kg *s.c.*** | | | | |
|---|---|---|---|---|
| Organ | Plasma | Brain | Liver | Lung |
| T½ | 0.8 | 3.6 | NA | 2.6 |
| AUC₍₀₋₂₄ₕ₎ ng/ml·h | 805 | 3188 | 493 | 17623 |
| AUC_{organ}/AUCₚₗₐₛₘₐ | NA | 4.0 | 0.6 | 21.9 |

### Example 18: Tissue distribution after repeat dosing

Compound (I) was dosed 15, 30 and 60 mg/kg p.o. to male and female NMRI mice for 14 days. Plasma, brain and liver were taken 2 h post last dose.

The ratio to brain remain in the same ballpark figures comparing single and repeat dosing. The ratio to liver is also the same as for single dose, except for the low dose to male mice. There is no single dose data from male mice.

**Table 20. Tissue distribution in mouse after repeat dosing**

| Compound (I) dosed *p.o.* to mice for 14 days. Samples taken 2 h post last dose | | | | | | | | |
|---|---|---|---|---|---|---|---|---|
| | Female | | | | Male | | | |
| Dose | 15 | 30 | 60 | 90 | 15 | 30 | 60 | 90 |

| **Concentrations in organs** | | | | | | | | |
|---|---|---|---|---|---|---|---|---|
| Plasma (ng/ml) | 390 | 1278 | 1997 | 3975 | 289 | 762 | 1420 | 4731 |
| Brain (ng/g) | 2641 | 7070 | 11498 | 17084 | 1966 | 4721 | 8084 | 21136 |
| Liver (ng/g) | 7161 | 22489 | 42042 | 63258 | 8221 | 12723 | 17735 | 75039 |

| **Target organ ratios** | | | | | | | | |
|---|---|---|---|---|---|---|---|---|
| Brain/plasma ratio | 7 | 6 | 6 | 4 | 7 | 6 | 6 | 4 |
| Liver/plasma ratio | 18 | 18 | 21 | 16 | 28 | 17 | 12 | 16 |

Compound (I) was dosed 5, 10 and 15 mg/kg s.c. to male and female Wistar rats for 14 days. Plasma, brain and liver were taken 2 h post last dose.

For rat, the ratio to brain is higher after repeat dosing as compared to single dose. This is true for liver as well. Compared to mouse, the ratio to liver is still much lower. Again this might be due to the higher metabolising capacity of the liver in rat. In contrast to mouse, male rats show higher concentration than females and the ratio to liver is consistently lower.

**Table 21. Tissue distribution in rats after repeat dosing**

| **Compound (I) dosed *s.c.* to rats for 14 days. Samples taken 2 h post last dose** | | | | | | | | |
|---|---|---|---|---|---|---|---|---|
| | **Female** | | | | **Male** | | | |
| Dose | 5 | 10 | 15 | 25 | 5 | 10 | 15 | 25 |

| **Concentrations in organs** | | | | | | | | |
|---|---|---|---|---|---|---|---|---|
| Plasma (ng/ml) | 67 | 149 | 281 | 412 | 77 | 242 | 398 | 512 |
| Brain (ng/g) | 468 | 1021 | 1578 | 2077 | 519 | 1384 | 1952 | 2580 |
| Liver (ng/g) | 319 | 738 | 1518 | 2311 | 259 | 737 | 921 | 1559 |

| **Target organ ratios** | | | | | | | | |
|---|---|---|---|---|---|---|---|---|
| Brain/plasma ratio | 7 | 7 | 6 | 5 | 7 | 6 | 5 | 5 |
| Liver/plasma ratio | 5 | 5 | 5 | 6 | 3 | 3 | 2 | 3 |

### Example 19: Metabolism - metabolic stability in liver microsomes

The metabolic stability of compound (I) was tested in liver microsomes from all in house available species at 1 and 5 µM. One metabolite of 16 mass units higher than parent, was found in all incubations, except with human liver microsomes. The metabolite showed the same retention time in all species, which indicates that it is most likely the same metabolite. compound (I) is relatively stable in all tested species except rat.

**Table 22. Metabolic stability of compound (I) in liver microsomes.**

| **Species** | **Stability at 1 µM** | **Stability at 5 µM** | **Metabolite** |
|---|---|---|---|
| Mouse | 89% | 100% | +16 |
| Rat | 40% | 61% | +16 |
| Guinea pig | 98% | 82% | +16 |
| Monkey | 79% | 88% | +16 |
| Minipig | 88% | 98% | +16 |
| Dog | 100% | 90% | +16 |
| Human | 94% | 93% | None |

### Example 20: Metabolism - metabolic stability in human hepatocytes

The stability of compound (I) was tested in human hepatocytes incubating for 120 minutes at 10 µM. The compound was stable in human hepatocytes.

### Example 21: Metabolism - CYP inhibition

Compound (I) was tested for CYP450 inhibition using a cocktail of selective substrates. The IC50 was determined as the concentration of compound (I), which inhibited the formation of metabolites by 50% (Table 23).CYP1A2 is the only one, which is significantly inhibited by compound (I). This might be addressed later in a clinical study.

**Table 23. CYP450 inhibition of compound (I).**

| **CYP** | **Substrate** | **Metabolite** | **IC50 of compound (I)** |
|---|---|---|---|
| 1A2 | Phenacetin | Paracetamol | 3.5 µM |
| 2C9 | Tolbutamide | 4-hydroxytolbutamide | >100 µM |
| 2C19 | Omeprazole | 5-hydroxyomeprazole | >100 µM |
| 2D6 | Bufuralol | 1'-hydroxybufuralol | 44 µM |
| 3A4 | Midazolam | 1'-hydroxymidazolam | >100 µM |

### Example 22: Metablism - in vivo metabolites

By quantifying compound (I) in rat urine, the recovery of parent compound in urine was estimated to 6-10% of the dose (0-24 h).

### Example 23: Protein binding

The plasma protein binding was tested in rat, mouse, dog and human plasma by equilibrium dialysis. The plasma protein binding of this compound is remarkably low (Table 24).

**Table 24. Protein binding by compound (I).**

| **Species** | **Test concentration** | **Result** |
|---|---|---|
| Rat | 2710 ng/ml | 36.5 % |
| Mouse | 2710 ng/ml | 25.3 % |
| Dog | 2710 ng/ml | 12.9 % |
| Human | 2710 ng/ml | 28.6 % |
| Rat | 81 ng/ml | 43.4 % |
| Rat | 271 ng/ml | 35.0 % |
| Rat | 813 ng/ml | 41.8 % |

### Example 24: Toxicology: Maximum tolerated dose and repeat dosing

Compound (I) administered by p.o. (non-GLP) in female mice for 5 days at the following dose levels; *study 1*: 3, 5, 15 and *study 2*: 30, 60 mg/kg. produced an increase in activity (sedation and stereotypic behaviour) but no significant changes in bodyweight.

Compound (I) administered p.o. (non-GLP) in female rats for 5 days at the following dose levels; 30, 60, 90 mg/kg resulted in increased activity but no changes in body weight gain were observed. Since the bioavailability in rats is low, the following studies were performed using *s.c.* dosing.in rats (~ 100% bioavailability).

In a 14-day repeat-dose study 4 groups of 8 male and 8 female NMRI mice were treated daily with compound (I) at doses of 15, 30, and 60 mg/kg p.o. (Groups 2, 3 and 4), or vehicle, 5% glucose s.c. (Group 1). A satellite group 90mg/kg p.o. (group 5) was conducted accordingly to increase the exposure and efficacy.

Compound (I) dosed at 60 mg/kg (Group 4), produced an increase in activity in both male and female, significant decrease in body weight in the female group and a significant decrease in relative liver weight in both sexes. The 90 mg/kg (Group 5) produced an increase in activity in both male and female, significant decrease in body weight and in relative liver weight in both sexes. At termination no treatment-related macroscopic findings were seen in male or female.

### Conclusion

Male and female mice treated p.o. for 14 days at dose levels from 15 up to 90 mg/kg/day showed treatment related significant changes in body weight and in relative liver weight, but no treatment-related macroscopic findings were seen in male or female.

In a 14-day repeat-dose study 4 groups of 4 male and 4 female Wistar rats were treated daily with either compound (I) at doses of 5, 10 and 15 mg/kg s.c. (Groups 2, 3 and 4), or vehicle, 5% glucose s.c. (Group 1). A satellite group 25mg/kg s.c. (group5) was conducted accordingly to increase the exposure and efficacy.

Compound (I) dosed at 15mg/kg (Group 4), produced an increased activity in both males and females. No changes in body weight gain were observed in the female rats, but a tendency to increased bodyweight in the group 4 males. At termination no treatment-related macroscopic findings were seen, but the relative liver weights were reduced in group 4 females and increased in group 4 males. Group 5 (25 mg/kg), produced an increased activity in both male and female and a minimal decreased body weight. No changes in liver weight were seen in group 5.

### Conclusion

Male and female Wistar rats were treated s.c. for 14 days, at dose levels from 5 to 25 mg/kg/day. The high dose group (25 mg/kg s.c.) showed minimal decreased body weight but increased activity in both male and female rats.

Compound (I) was tested for genotoxicity in the Ames screening test using Salmonella typhimurium strains TA 100 and TA 98. Compound (I) was tested at dose levels of 1.6 to 5000 µg/plate in the presence or absence of S-9 mix. It was concluded that compound (I) did not show any evidence of mutagenic activity in this test.

### Example 25: Induction of erection in rats

Compound (I) was administered to rats at 1mg/kg, leading to improved erectile function as outlined in Figure 25. Vehicle and compound (I) were administered after establishing the maximal (10 Hz, 1 ms, 6 V) and submaximal (submax) responses (10 Hz, 1 ms, 0.6 - 1.55 V) to cavernous nerve stimulation for 30 s. Submaximal stimulation was repeated 3, 13, 23, and 33 min after drug or vehicle administration (Figure 27). Compound (I) was found to induce erection in both young and adult rats.

### Example 26: Dose dependent frequency of erectile events in rats

Compound (I) was administered intravenously to rats in dosages of 0.001, 0.01, 0.1, and 1.0 mg/kg, and the number of spontaneous erections was measured. The number of events increased with increasing dosage from 0.001 to 0.1 mg/kg, but decreased abruptly at a dose of 1.0 mg/kg (Figure 26). Thus, compound (I) is more efficient at inducing erection in rats at low concentrations.

### Example 27: Effect of compound (I) on corpus cavernosum contractility

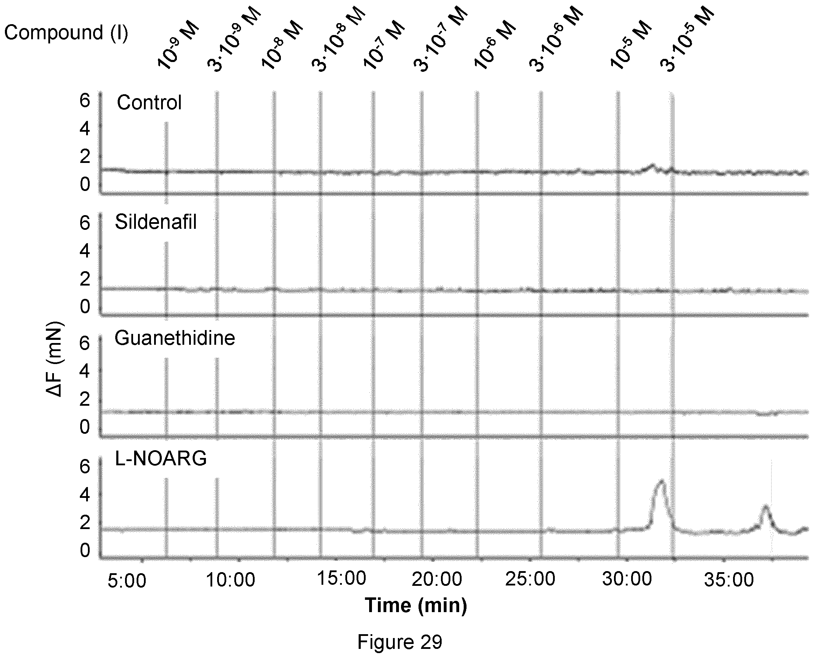

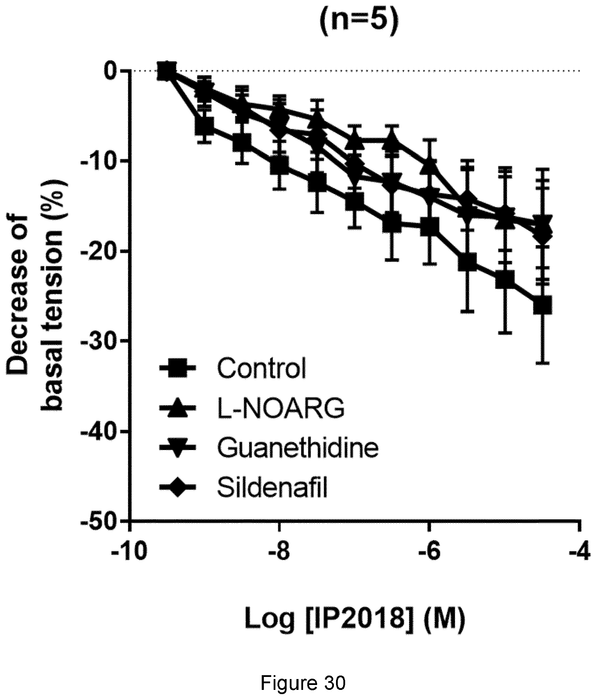

In resting corpus cavernosum strips, increasing concentrations of compound (I) (10⁻⁹-3×10⁻⁵ M) were added and produced only small relaxations (Figure 29). Only occasionally, contraction was observed at the highest concentration (3×10⁻⁵ M) of compound (I). The relaxations were unaltered in the presence of a nitric oxide synthase inhibitor, L-NOARG (10⁻⁴ M) and an inhibitor of phosphodiesterase type 5 inhibitor, sildenafil, while treatment with guanethidine leading to depletion of noradrenaline inhibited contractions, but did not change the relaxations (Figure 29, 30).

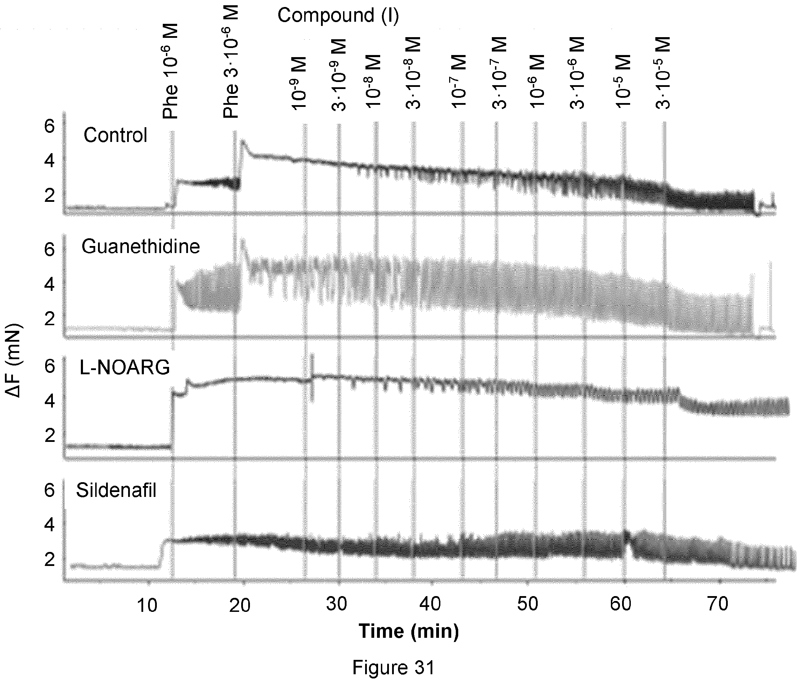

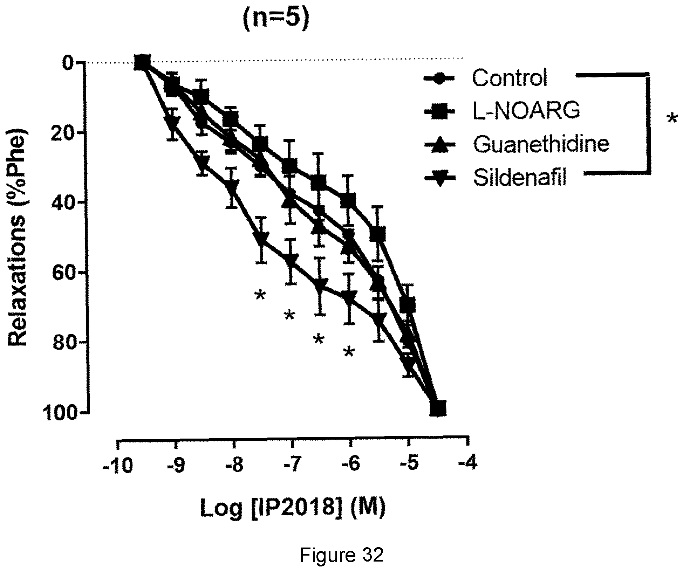

In strips contracted with phenylephrine (10⁻⁶ M), compound (I) in low concentrations induced rapid relaxations followed by contraction, while concentration-dependent relaxations were observed at higher concentrations (Figure 31). Sildenafil markedly enhanced these relaxations, while a nitric oxide synthase inhibitor, L-NOARG (3×10⁻⁵ M) tended to inhibit these relaxations (Figure 32). These findings suggest that the low concentrations of compound (I) through nitric oxide release leads to relaxations of rat corpus cavernosum tissue, and that this effect may contribute to the erectile effect of the compound. These results strongly suggest that compound (I) may be administered locally to or near the penis, such as transdermally or intracavernous.

### Example 28: Effect of compound (I) on penile flow in mice

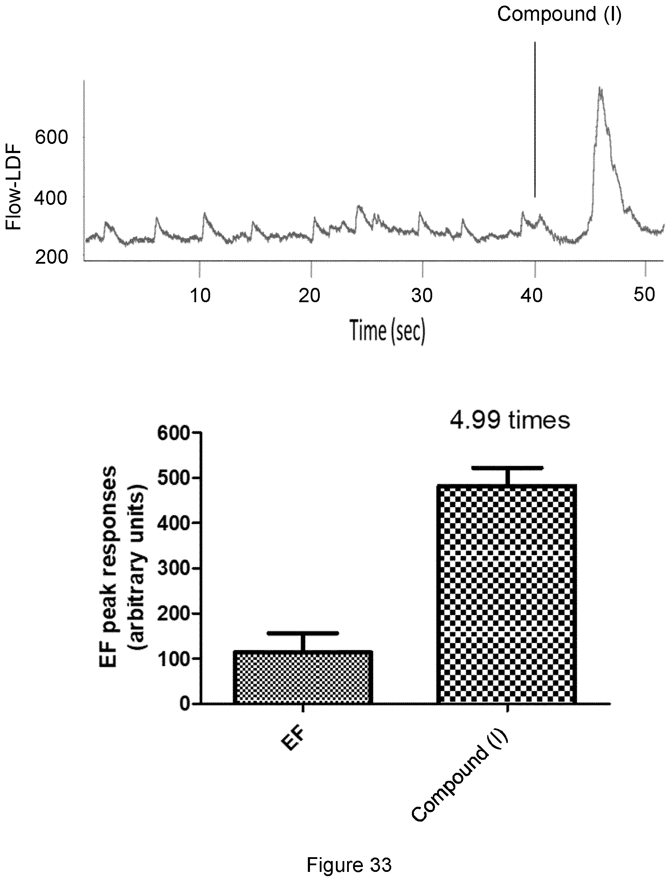

A laser doppler flow probe was positioned in situ in mice erectile tissue for measurement of penile basal flow in the absence and the presence of compound (I). Penile flow markedly increased by infusion of compound (I) (1 mg/kg) (Figure 33). These results strongly suggest that compound (I) may be administered locally to or near the penis, such as transdermally or intracavernous.

## Claims

1. A compound of formula (I), 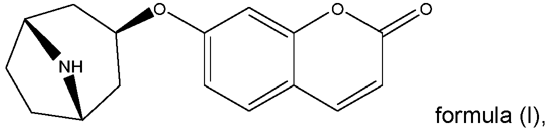 or a pharmaceutically acceptable salt thereof, for use in the treatment, prevention, or alleviation of a combination of erectile dysfunction and depression in a mammal.

2. The compound for use according to claim 1, wherein the erectile dysfunction is treatment-emergent erectile dysfunction.

3. The compound for use according to any one of claims 1 and 2, wherein the erectile dysfunction is caused by depression.

4. The compound for use according to claim 2, or a pharmaceutically acceptable salt thereof, wherein the treatment-emergent erectile dysfunction is an adverse effect originating from treatment of a medicament.

5. The compound for use according to claim 4, wherein the medicament is selected from the group consisting of antidepressants, NSAIDs, finasteride, antiepileptics and neuroleptics.

6. The compound for use according to any one of claims 4 and 5, wherein the erectile dysfunction is treatment-emergent erectile dysfunction caused by the treatment by an antidepressant medicament.

7. The compound for use according to any one of claims 4 to 6, wherein the erectile dysfunction is treatment-emergent erectile dysfunction caused by the treatment of depression by an antidepressant medicament.

8. The compound for use according to claim 1, wherein the depression is caused by erectile dysfunction.

9. The compound for use according to claim 1, wherein the mammal is a human.

10. The compound for use according to claim 9, wherein the human is male.

11. The compound for use according to any one of claims 1 to 10, wherein the subject is a male below 40 years of age, such as below 35 years of age, such as below 30 years of age, such as below 25 years of age, such as below 20 years of age.

12. The compound for use according to any one of claims 1 to 10, wherein the subject is a male, such as a male over the age of 20, such as a male over the age of 25, such as a male over the age of 30, such as a male over the age of 35, such as a male over the age of 40, such as a male over the age of 45, such as a male over the age of 50, such as a male over the age of 55, such as a male over the age of 60, such as a male over the age of 65, such as a male over the age of 70, such as a male over the age of 75.

13. The compound for use according to any one of claims 1 to 12, wherein the mammal is not simultaneously treated with other anxiolytic and/or antidepressive medication.

14. The compound for use according to any one of claims 1 to 13, wherein the compound is administered systemically.

15. The compound for use according to claim 14, wherein the compound is administered orally.

## Patentansprüche

1. Verbindung von Formel (I) 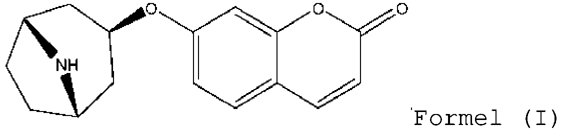 oder pharmazeutisch annehmbares Salz davon zur Verwendung bei der Behandlung, Vorbeugung oder Linderung von einer Kombination aus erektiler Dysfunktion und Depression bei einem Säugetier.

2. Verbindung zur Verwendung nach Anspruch 1, wobei die erektile Dysfunktion eine behandlungsbedingte erektile Dysfunktion ist.

3. Verbindung zur Verwendung nach einem der Ansprüche 1 und 2, wobei die erektile Dysfunktion durch Depression verursacht wird.

4. Verbindung zur Verwendung nach Anspruch 2 oder pharmazeutisch annehmbares Salz davon, wobei die behandlungsbedingte erektile Dysfunktion eine nachteilige Wirkung aus der Behandlung mit einem Medikament ist.

5. Verbindung zur Verwendung nach Anspruch 4, wobei das Medikament aus der Gruppe bestehend aus Antidepressiva, NSAIDs, Finasterid, Antiepileptika und Neuroleptika ausgewählt ist.

6. Verbindung zur Verwendung nach einem der Ansprüche 4 und 5, wobei die erektile Dysfunktion eine behandlungsbedingte erektile Dysfunktion ist, die durch die Behandlung mit einem Antidepressivum verursacht wird.

7. Verbindung zur Verwendung nach einem der Ansprüche 4 bis 6, wobei die erektile Dysfunktion eine behandlungsbedingte erektile Dysfunktion ist, die durch die Behandlung von Depressionen mit einem Antidepressivum verursacht wird.

8. Verbindung zur Verwendung nach Anspruch 1, wobei die Depression durch erektile Dysfunktion verursacht wird.

9. Verbindung zur Verwendung nach Anspruch 1, wobei das Säugetier ein Mensch ist.

10. Verbindung zur Verwendung nach Anspruch 9, wobei der Mensch ein Mann ist.

11. Verbindung zur Verwendung nach einem der Ansprüche 1 bis 10, wobei das Subjekt ein Mann unter 40 Jahren, wie etwa unter 35 Jahren, wie etwa unter 30 Jahren, wie etwa unter 25 Jahren, wie etwa unter 20 Jahren ist.

12. Verbindung zur Verwendung nach einem der Ansprüche 1 bis 10, wobei das Subjekt ein Mann ist, wie etwa ein Mann über 20, wie etwa ein Mann über 25, wie etwa ein Mann über 30, wie etwa ein Mann über 35, wie etwa ein Mann über 40, wie etwa ein Mann über 45, wie etwa ein Mann über 50, wie etwa ein Mann über 55, wie etwa ein Mann über 60, wie etwa ein Mann über 65, wie etwa ein Mann über 70, wie etwa ein Mann über 75.

13. Verbindung zur Verwendung nach einem der Ansprüche 1 bis 12, wobei das Säugetier nicht gleichzeitig mit anderen anxiolytischen Medikamenten und/oder Antidepressiva behandelt wird.

14. Verbindung zur Verwendung nach einem der Ansprüche 1 bis 13, wobei die Verbindung systemisch verabreicht wird.

15. Verbindung zur Verwendung nach Anspruch 14, wobei die Verbindung oral verabreicht wird.

## Revendications

1. Composé de formule (I), 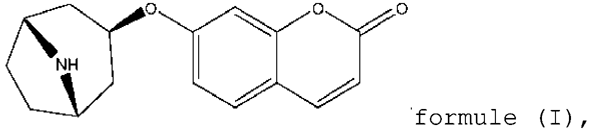 ou un sel pharmaceutiquement acceptable de celui-ci, pour une utilisation dans le traitement, la prévention ou le soulagement d'une combinaison de dysfonction érectile et de dépression chez un mammifère.

2. Composé destiné à être utilisé selon la revendication 1, dans lequel la dysfonction érectile est une dysfonction érectile émergeant d'un traitement.

3. Composé destiné à être utilisé selon l'une quelconque des revendications 1 et 2, dans lequel la dysfonction érectile est provoquée par la dépression.

4. Composé destiné à être utilisé selon la revendication 2, ou un sel pharmaceutiquement acceptable de celui-ci, dans lequel la dysfonction érectile émergeant d'un traitement est un effet indésirable provenant du traitement d'un médicament.

5. Composé destiné à être utilisé selon la revendication 4, dans lequel le médicament est choisi dans le groupe composé des antidépresseurs, des AINS, du finastéride, des antiépileptiques et des neuroleptiques.

6. Composé destiné à être utilisé selon l'une quelconque des revendications 4 et 5, dans lequel la dysfonction érectile est une dysfonction érectile émergeant d'un traitement provoqué par le traitement par un médicament antidépresseur.

7. Composé destiné à être utilisé selon l'une quelconque des revendications 4 à 6, dans lequel la dysfonction érectile est une dysfonction érectile émergeant d'un traitement provoqué par le traitement de la dépression par un médicament antidépresseur.

8. Composé destiné à être utilisé selon la revendication 1, dans lequel la dépression est provoquée par la dysfonction érectile.

9. Composé destiné à être utilisé selon la revendication 1, dans lequel le mammifère est un être humain.

10. Composé destiné à être utilisé selon la revendication 9, dans lequel l'être humain est un homme.

11. Composé destiné à être utilisé selon l'une quelconque des revendications 1 à 10, dans lequel le sujet est un homme de moins de 40 ans, tel que de moins de 35 ans, tel que de moins de 30 ans, tel que de moins de 25 ans, tel que de moins de 20 ans.

12. Composé destiné à être utilisé selon l'une quelconque des revendications 1 à 10, dans lequel le sujet est un homme, tel qu'un homme de plus de 20 ans, tel qu'un homme de plus de 25 ans, tel qu'un homme de plus de 30 ans, tel qu'un homme de plus de 35 ans, tel qu'un homme de plus de 40 ans, tel qu'un homme de plus de 45 ans, tel qu'un homme de plus de 50 ans, tel qu'un homme de plus de 55 ans, tel qu'un homme de plus de 60 ans, tel qu'un homme de plus de 65 ans, tel qu'un homme de plus de 70 ans, tel qu'un homme de plus de 75 ans.

13. Composé destiné à être utilisé selon l'une quelconque des revendications 1 à 12, dans lequel le mammifère n'est pas traité simultanément avec un autre médicament anxiolytique et/ou antidépresseur.

14. Composé destiné à être utilisé selon l'une quelconque des revendications 1 à 13, dans lequel le composé est administré par voie systémique.

15. Composé destiné à être utilisé selon la revendication 14, dans lequel le composé est administré par voie orale.
